(19) Europäisches Patentamt
European Patent Office
Office européen des brevets

(11) **EP 2 842 962 A1**

(12) **EUROPEAN PATENT APPLICATION**

(43) Date of publication:
**04.03.2015 Bulletin 2015/10**

(51) Int Cl.:
***C07K 1/22*** *(2006.01)* ***G01N 33/68*** *(2006.01)*

(21) Application number: **13182421.1**

(22) Date of filing: **30.08.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Ludwig-Maximilians-Universität München**
**80539 München (DE)**

(72) Inventors:
- **Shi, Song**
  **81377 München (DE)**
- **Kretzschmar, Hans A.**
  **82319 Starnberg (DE)**
- **Giese, Armin**
  **81377 München (DE)**

(74) Representative: **Leissler-Gerstl, Gabriele**
**Hoefer & Partner**
**Patentanwälte**
**Pilgersheimer Strasse 20**
**81543 München (DE)**

(54) **Method for the isolation of recombinant prion protein and the use thereof**

(57) The present invention is concerned with imidazole-cascade elution (ICE), which is a method for isolating distinct fractions from a source of protein. Specifically, it is concerned with the isolation of a specific fraction ($rPrP^{ox}$) from purified recombinant prion protein (rPrP), the use of the novel protein ($rPrP^{ox}$) in detecting scrapie prion protein ($PrP^{Sc}$) in food samples or in samples obtained from mammals, such as humans, and a kit comprising the rPrP for use for detecting $PrP^{Sc}$.

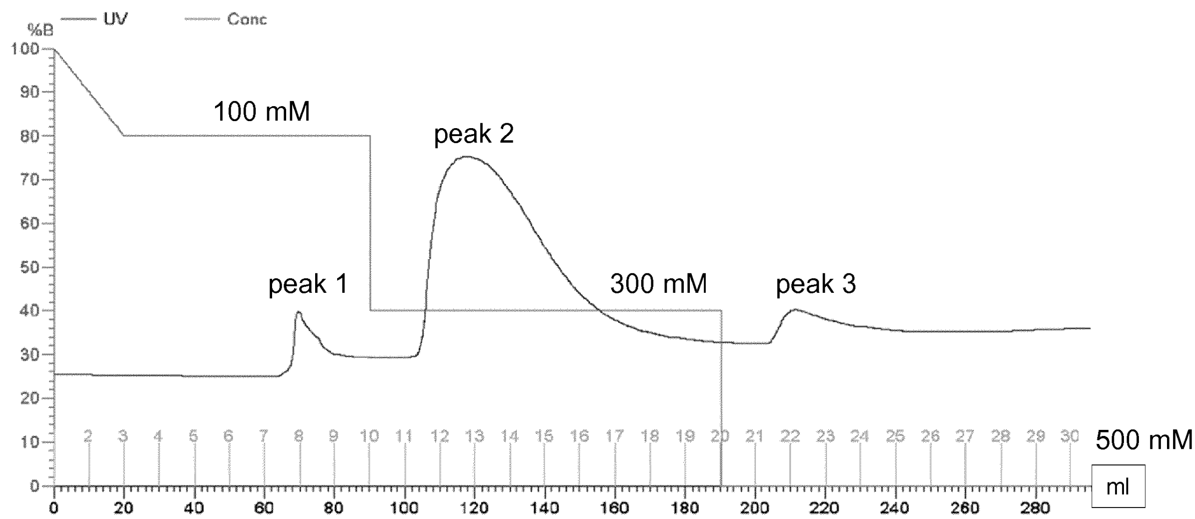


**Figure 13**

EP 2 842 962 A1

SEQUENCE LISTING

<110> Ludwig-Maximilians-UniversitÃ¤t MÃ¼nchen

<120> Method for the isolation of recombinant prion protein and the use thereof

<130> LMU130801PEP

<160> 5

<170> BiSSAP 1.2

<210> 1
<211> 624
<212> DNA
<213> Artificial Sequence

<220>
<221> source
<222> 1..624
<223> /mol_type="unassigned DNA"
/note="CDS of prion protein"
/organism="Artificial Sequence"

<400> 1
aagaagcgcc cgaagcctgg aggatggaac actgggggca gccgataccc ggggcagggc 60

agccctggag gcaaccgcta cccacctcag ggcggtggtg gctgggggca gcctcatggt 120

ggtggctggg ggcagcctca tggtggtggc tgggggcagc cccatggtgg tggctgggga 180

cagcctcatg gtggtggctg gggtcaagga ggtggcaccc acagtcagtg gaacaagccg 240

agtaagccaa aaaccaacat gaagcacatg gctggtgctg cagcagctgg ggcagtggtg 300

gggggccttg gcggctacat gctgggaagt gccatgagca ggcccatcat acatttcggc 360

agtgactatg aggaccgtta ctatcgtgaa aacatgcacc gttaccccaa ccaagtgtac 420

tacaggccca tggatgagta cagcaaccag aacaactttg tgcacgactg cgtcaatatc 480

acaatcaagc agcacacggt caccacaacc accaaggggg agaacttcac cgagaccgac 540

gttaagatga tggagcgcgt ggttgagcag atgtgtatca cccagtacga gagggaatct 600

caggcctatt accagagagg atcg 624

<210> 2
<211> 208
<212> PRT
<213> Artificial Sequence

<220>
<223> recombinant prion protein

<400> 2
Lys Lys Arg Pro Lys Pro Gly Gly Trp Asn Thr Gly Gly Ser Arg Tyr
1 5 10 15
Pro Gly Gln Gly Ser Pro Gly Gly Asn Arg Tyr Pro Pro Gln Gly Gly
20 25 30
Gly Gly Trp Gly Gln Pro His Gly Gly Gly Trp Gly Gln Pro His Gly

```
        35                  40                  45
Gly Gly Trp Gly Gln Pro His Gly Gly Gly Trp Gly Gln Pro His Gly
    50                  55                  60
Gly Gly Trp Gly Gln Gly Gly Gly Thr His Ser Gln Trp Asn Lys Pro
65                  70                  75                  80
Ser Lys Pro Lys Thr Asn Met Lys His Met Ala Gly Ala Ala Ala Ala
                85                  90                  95
Gly Ala Val Val Gly Gly Leu Gly Gly Tyr Met Leu Gly Ser Ala Met
            100                 105                 110
Ser Arg Pro Ile Ile His Phe Gly Ser Asp Tyr Glu Asp Arg Tyr Tyr
        115                 120                 125
Arg Glu Asn Met His Arg Tyr Pro Asn Gln Val Tyr Tyr Arg Pro Met
    130                 135                 140
Asp Glu Tyr Ser Asn Gln Asn Asn Phe Val His Asp Cys Val Asn Ile
145                 150                 155                 160
Thr Ile Lys Gln His Thr Val Thr Thr Thr Thr Lys Gly Glu Asn Phe
                165                 170                 175
Thr Glu Thr Asp Val Lys Met Met Glu Arg Val Val Glu Gln Met Cys
            180                 185                 190
Ile Thr Gln Tyr Glu Arg Glu Ser Gln Ala Tyr Tyr Gln Arg Gly Ser
        195                 200                 205
```

<210> 3
<211> 2415
<212> DNA
<213> Homo sapiens

<220>
<221> source
<222> 1..2415
<223> /mol_type="unassigned DNA"
/note="GenBank entry M13899.1"
/organism="Homo sapiens"

<400> 3

```
cggcgccgcg agcttctcct ctcctcacga ccgaggcaga gcagtcatta tggcgaacct      60

tggctgctgg atgctggttc tctttgtggc cacatggagt gacctgggcc tctgcaagaa     120

gcgcccgaag cctggaggat ggaacactgg gggcagccga tacccggggc agggcagccc     180

tggaggcaac cgctacccac ctcagggcgg tggtggctgg gggcagcctc atggtggtgg     240

ctgggggcag cctcatggtg gtggctgggg gcagccccat ggtggtggct ggggacagcc     300

tcatggtggt ggctggggtc aaggaggtgg cacccacagt cagtggaaca agccgagtaa     360

gccaaaaacc aacatgaagc acatggctgg tgctgcagca gctggggcag tggtgggggg     420

ccttggcggc tacatgctgg gaagtgccat gagcaggccc atcatacatt tcggcagtga     480

ctatgaggac cgttactatc gtgaaaacat gcaccgttac cccaaccaag tgtactacag     540

gcccatggat gagtacagca accagaacaa ctttgtgcac gactgcgtca atatcacaat     600

caagcagcac acggtcacca caaccaccaa gggggagaac ttcaccgaga ccgacgttaa     660

gatgatggag cgcgtggttg agcagatgtg tatcacccag tacgagaggg aatctcaggc     720

ctattaccag agaggatcga gcatggtcct cttctcctct ccacctgtga tcctcctgat     780
```

ctctttcctc atcttcctga tagtgggatg aggaaggtct tcctgttttc accatctttc 840

taatcttttt ccagcttgag ggaggcggta tccacctgca gccctttttag tggtggtgtc 900

tcactctttc ttctctcttt gtcccggata ggctaatcaa taccettggc actgatgggc 960

actggaaaac atagagtaga cctgagatgc tggtcaagcc ccctttgatt gagttcatca 1020

tgagccgttg ctaatgccag gccagtaaaa gtataacagc aaataaccat tggttaatct 1080

ggacttattt ttggacttag tgcaacaggt tgaggctaaa acaaatctca gaacagtctg 1140

aaataccttt gcctggatac ctctggctcc ttcagcagct agagctcagt atactaatgc 1200

cctatcttag tagagatttc atagctattt agagatattt tccattttaa gaaaacccga 1260

caacatttct gccaggtttg ttaggaggcc acatgatact tattcaaaaa aatcctagag 1320

attcttagct cttgggatgc aggctcagcc cgctggagca tgagctctgt gtgtaccgag 1380

aactggggtg atgttttact tttcacagta tgggctacac agcagctgtt caacaagagt 1440

aaatattgtc acaacactga acctctggct agaggacata ttcacagtga acataactgt 1500

aacatatatg aaaggcttct gggacttgaa atcaaatgtt tgggaatggt gcccttggag 1560

gcaacctccc attttagatg tttaaaggac cctatatgtg gcattccttt ctttaaacta 1620

taggtaatta aggcagctga aaagtaaatt gccttctaga cactgaaggc aaatctcctt 1680

tgtccattta cctggaaacc agaatgattt tgacatacag gagagctgca gttgtgaaag 1740

caccatcatc atagaggatg atgtaattaa aaaatggtca gtgtgcaaag aaaagaactg 1800

cttgcatttc tttatttctg tctcataatt gtcaaaaacc agaattaggt caagttcata 1860

gtttctgtaa ttggcttttg aatcaaagaa tagggagaca atctaaaaaa tatcttaggt 1920

tggagatgac agaaatatga ttgatttgaa gtggaaaaag aaattctgtt aatgttaatt 1980

aaagtaaaat tattccctga attgtttgat attgtcacct agcagatatg tattactttt 2040

ctgcaatgtt attattggct tgcactttgt gagtatctat gtaaaaatat atatgtatat 2100

aaaatatata ttgcatagga cagacttagg agttttgttt agagcagtta acatctgaag 2160

tgtctaatgc attaactttt gtaaggtact gaatacttaa tatgtgggaa acccttttgc 2220

gtggtcctta ggcttacaat gtgcactgaa tcgtttcatg taagaatcca aagtggacac 2280

cattaacagg tctttgaaat atgcatgtac tttatatttt ctatatttgt aactttgcat 2340

gttcttgttt tgttatataa aaaaattgta aatgtttaat atctgactga aattaaacga 2400

gcgaagatga gcacc 2415

<210> 4
<211> 253
<212> PRT
<213> Homo sapiens

<220>

```
<223> GenBank entry AAA60182.1

<400> 4
Met Ala Asn Leu Gly Cys Trp Met Leu Val Leu Phe Val Ala Thr Trp
1               5                   10                  15
Ser Asp Leu Gly Leu Cys Lys Lys Arg Pro Lys Pro Gly Gly Trp Asn
            20                  25                  30
Thr Gly Gly Ser Arg Tyr Pro Gly Gln Gly Ser Pro Gly Gly Asn Arg
        35                  40                  45
Tyr Pro Pro Gln Gly Gly Gly Gly Trp Gly Gln Pro His Gly Gly Gly
    50                  55                  60
Trp Gly Gln Pro His Gly Gly Gly Trp Gly Gln Pro His Gly Gly Gly
65                  70                  75                  80
Trp Gly Gln Pro His Gly Gly Gly Trp Gly Gln Gly Gly Gly Thr His
                85                  90                  95
Ser Gln Trp Asn Lys Pro Ser Lys Pro Lys Thr Asn Met Lys His Met
            100                 105                 110
Ala Gly Ala Ala Ala Ala Gly Ala Val Val Gly Gly Leu Gly Gly Tyr
        115                 120                 125
Met Leu Gly Ser Ala Met Ser Arg Pro Ile Ile His Phe Gly Ser Asp
    130                 135                 140
Tyr Glu Asp Arg Tyr Tyr Arg Glu Asn Met His Arg Tyr Pro Asn Gln
145                 150                 155                 160
Val Tyr Tyr Arg Pro Met Asp Glu Tyr Ser Asn Gln Asn Asn Phe Val
                165                 170                 175
His Asp Cys Val Asn Ile Thr Ile Lys Gln His Thr Val Thr Thr Thr
            180                 185                 190
Thr Lys Gly Glu Asn Phe Thr Glu Thr Asp Val Lys Met Met Glu Arg
        195                 200                 205
Val Val Glu Gln Met Cys Ile Thr Gln Tyr Glu Arg Glu Ser Gln Ala
    210                 215                 220
Tyr Tyr Gln Arg Gly Ser Ser Met Val Leu Phe Ser Ser Pro Pro Val
225                 230                 235                 240
Ile Leu Leu Ile Ser Phe Leu Ile Phe Leu Ile Val Gly
                245                 250

<210> 5
<211> 8
<212> PRT
<213> Artificial Sequence

<220>
<223> octarepeat

<400> 5
Gln Pro His Gly Gly Gly Trp Gly
1               5
```

## Description

### Field of the invention

The present invention is concerned with Imidazole-cascade elution (ICE), which is a method for isolating distinct fractions from a source of self-aggregating protein. Specifically, it is concerned with the isolation of a highly purified recombinant prion protein (rPrP), the use of the recombinant protein in detecting scrapie prion protein ($PrP^{Sc}$) in food samples or in samples obtained from mammals, such as humans, and a kit comprising highly purified rPrP for use for detecting $PrP^{Sc}$.

### Background

Transmissible spongiform encephalopathies (TSEs), also known as "prion diseases" are a group of transmissible neurodegenerative diseases affecting primarily the central nervous system (CNS) of many animals, including humans. TSEs of humans include sporadic and genetic Creutzfeldt-Jakob disease, variant Creutzfeldt-Jakob disease (vCJD), a human disorder caused by the infectious agent of bovine spongiform encephalopathy (BSE), Gerstmann-Sträussler-Scheinker syndrome, fatal familial insomnia and kuru. TSEs of other mammals include BSE of cattle, scrapie of sheep and goats, feline spongiform encephalopathy (FSE) of cats, transmissible mink encephalopathy (TME) of minks, exotic ungulate encephalopathy (EUE) of Nyala and Greater Kudu, as well as chronic wasting disease (CWD) of deer and elk.

The infectious agent of TSEs is called "prion", which is derived from "proteinaceous" and "infectious". A prion is the infectious agent causing prion diseases. The only known constituent of the prion is the prion protein in a misfolded protein state ($PrP^{Sc}$) compared to the properly folded common protein ($PrP^{C}$). The protein that prions are made of (PrP) is found throughout the body, even in healthy people and animals. However, PrP found in infectious material has a different structure and one part of it is partially resistant to proteases, the enzymes in the body that can normally break down proteins. The normal form of the protein is called $PrP^{C}$, while the infectious form is called $PrP^{Sc}$ - the C refers to 'cellular' PrP, while the Sc refers to 'scrapie', a prion disease occurring in sheep.

Prions propagate by transmitting a misfolded protein state. When a prion enters a healthy organism, it induces existing, properly folded proteins to convert into the disease-associated, prion form; prion acts as a template to guide the misfolding of more proteins into prion form. These newly formed prions can then go on to convert more proteins themselves; this triggers a chain reaction that produces large amounts of the prion form. All known prions induce the formation of an amyloid fold, in which the protein polymerises into an aggregate consisting of tightly packed beta sheets. Amyloid aggregates are fibrils, growing at their ends, and replicating when breakage causes two growing ends to become four growing ends. This altered structure is extremely stable and accumulates in infected tissue.

Misfolding and aggregation of cellular proteins underlies a number of human neurodegenerative diseases in addition to TSEs, such as Alzheimer's, Parkinson's, and AA amyloidosis. Type 2 diabetes is yet another disease whose pathogenesis involves ordered protein aggregation. The propagation theory described for PrP may also apply to amyloid formation in other protein misfolding disorders (PMDs).

In fact, protein conformation changes associated with the pathogenesis of most PMDs result in the formation of abnormal proteins that are rich in $\beta$-sheet structure, partially resistant to proteolysis, and have a high tendency to form larger-order aggregates, similar to $PrP^{Sc}$. Amyloid formation depends on the slow interaction of misfolded protein monomers to form oligomeric nuclei, around which a faster phase of elongation takes place. The ability of oligomeric species to seed their own growth is analogous to the self-propagating activity of prions.

From a molecular standpoint, all PMDs can be considered "infectious" (self-propagating) in the sense that misfolded and aggregated proteins act as templates for the recruitment of native proteins. To stop the propagation of the misfolded protein, it is beneficial to carry out a rapid and reliable test of detecting the misfolded protein in a very early stage of the disease before the symptoms caused by the aggregations are manifest. Upon detection, a successful therapeutic approach may be designed.

TSEs, however, are unique and lethal diseases in that their aetiology may be genetic, sporadic or infectious via ingestion of infected foodstuffs and/or via iatrogenic means (e.g. blood transfusion). Most TSEs are sporadic and occur in an animal with no prion protein mutation. Inherited TSE occurs in humans carrying a rare mutant prion allele, which expresses prion proteins that eventually lead to the disease-causing conformation. Transmission occurs when healthy animals consume contaminated tissues from others with the disease. Thus, TSEs carry risks for both human medicine and agriculture, as well as in the related industries. It is important to provide a rapid and reliable diagnostic method to detect the infectious agent, the prion.

There continues to be a very practical problem with the detection of misfolded proteins and thus the diagnosis of PMD. This is specifically the case with prion diseases, including BSE and CJD, as they have an incubation period of months to decades during which there are no symptoms, even though the pathway of converting the normal brain PrP protein into the toxic, disease-related $PrP^{Sc}$ form has started. At present, there is virtually no way to detect $PrP^{Sc}$ reliably except by examining brain tissue from either biopsy (e.g. from neurosurgery) or autopsy (after death) using neuropathological and immunohistochemical methods. Accumulation of the abnormally folded $PrP^{Sc}$ form of the $PrP^{C}$ is a characteristic of the disease, but in accessible body fluids like blood or urine it is present at very low levels. Researchers have tried to develop methods to detect/measure $PrP^{Sc}$, but there are still no fully accepted methods for use in materials such as blood.

It is well known that $PrP^{Sc}$ can propagate autocatalytically *in vitro* by converting cellular prion protein ($PrP^{c}$) and recombinant prion protein (rPrP) to pathogenic forms. Based on this property, a novel amplification system, termed quaking-induced conversion (QuIC), was reported in 2008 (Atarashi R, Wilham JM, Christensen L et al., Nat Methods 2008;5:211-212; [1]). By mixing samples containing $PrP^{Sc}$ with rPrP in solution, $PrP^{Sc}$ could induce misfolding of rPrP and thus multiply efficiently *in vitro.* Thus small amounts of $PrP^{Sc}$, which were undetectable by using conventional methods, could propagate to reach detectable levels in a reasonable time period. The novel real-time QuIC (RT-QuIC) was developed by using an auto-read set-up to monitor the prion propagating procedure with fluorescence so that it allows testing multiple samples on one 96-well plate (Wilham JM et al.; PLoS 2010;6(12) [2]; Atarashi R et al.; Nature Medicine 2011; 17(1) [3]; Atarashi et al.; Prion 2011; 5:150-153 [4]; Orru et al; Protein Eng Des Sel. 2009; 22: 515-521; [5]).

It is very important for the diagnostic method to be very reliable. However, it was found by the inventors that diagnostic methods using RT-QuIC are prone to false negatives, and false positives. A false-positive result in a sample obtained from a domestic animal will most probably lead to the culling of a whole herd to control and/or prevent the outbreak of the infection. This is ethically and economically disastrous. A false-negative result will leave the disease undetected and lead to an uncontrolled propagation of the disease. Thus, it was an objective to provide a diagnostic agent and a method to reliably detect $PrP^{Sc}$ in a sample.

Following the conventional methods of purification of rPrP [2,3] and applying said "purified" rPrP in RT-QuIC for detecting $PrP^{Sc}$ in cerebrospinal fluid (CSF) from human sporadic CJD (sCJD) patients, however, led to several reactions with false negativity and low activity (see Fig. 1A and B). Increasing the reaction temperature was thought to increase the protein activity, but this caused false positives (see Fig. 1 C), which are not acceptable for the diagnosis of lethal diseases.

It is furthermore important for the diagnostic method to be efficient, as reliable results need to be obtained within a short time, i.e. within hours rather than days or weeks. As the number of samples obtained from sources such as foodstuff, humans or animals, or medical equipment, which will need to be checked routinely for presence of misfolded protein is high, the diagnostic method also needs to be applicable in high-throughput scale and therefore needs to be rapid, safe and cost-efficient. To allow for a rapid and cost-effective test result a diagnostic agent that is very active is necessary as well. The diagnostic agent is a rPrP that is converted in the presence of infectious PrP in a sample, i.e. a form of rPrP that is "misfolded" if $PrP^{Sc}$ is present. The diagnostic agent currently used in conventional methods is recombinant $rPrP^{c}$, which is very expensive. It was therefore a further objective of the present invention, to provide a novel rPrP, which is more active compared to the currently used rPrP, and therefore allows a more cost-efficient and rapid diagnostic method.

The above mentioned problems had never been taken into consideration for the methodology of the RT-QuIC system. Thus, it was an objective of the present invention to establish a method for diagnosing TSEs, which is more reliable and more efficient than conventional methods. It was further surprisingly found that the current protocols used for RT-QuIC provided unreliable results because of the nature of the rPrP solution used. Thus it was a further objective for the present invention to provide an improved rPrP, which can be used in RT-QuIC and allows for a more reliable and effective diagnosis of the infectious agent, $PrP^{Sc}$, in samples.

Thus, it was a further objective of the present invention to establish a method for providing an improved and more active form of rPrP for stabilizing the RT-QuIC system. Since the ICE-prepared rPrP is stable and reliable, it can also be used as the substrate for other detection techniques for prion diagnosis and drug screening, including protein misfolding cyclic amplification (PMCA), amyloid seeding assay (ASA) and scanning for intensely fluorescent targets (SIFT) assay.

It was a further objective to provide a protein useful as a positive control, which is not biohazardous, and therefore does not require expensive safety measures when being handled and shipped.

## Summary of the present invention

These problems were solved by the method of the present invention, which is termed imidazole cascade elution (ICE). It was surprisingly found that an rPrP preparation used in known detection methods comprised a mixture of similar, slightly different proteins or protein variants, respectively, with slightly differing characteristics and that a method for detecting $PrP^{sc}$ in a sample in a reliable and fast manner can be provided by using an isolated variant of rPrP as diagnostic agent in a RT-QuIC test. Known rPrP preparations comprise a rPrP mixture which might be the reason for the unreliable results obtained with those preparations. The method of the present invention using ICE to separate this rPrP mixture into fractions comprising differing rPrP variants, allows obtaining a novel, purified, isolated rPrP. This novel rPrP, also called $rPrP^{ox}$ in the following, has much higher purity and activity and, therefore, can be used to diagnose prion disease in a short time. This novel rPrP variant of the present invention can be used in diagnostic methods, such as RT-QuIC, and yields more reliable results. Fewer random false-negative and false-positive results are obtained compared to conventional diagnostic methods. The new rPrP variant, $rPrP^{ox}$, due to its higher purity has higher activity. Thus, the method of the present invention using this new rPrP is also more efficient, as less rPrP is needed, due to the improved activity of the rPrP of the present invention. When using $rPrP^{ox}$ the infectious agent causing CJD and other prion diseases can be detected even in blood of the patient. Moreover, the $rPrP^{ox}$ variant of the present invention can be used in high-throughput methods.

Furthermore, a diagnostic method for detecting prion diseases based on the known RT-QuIC technique in a short time is provided. This method provides reliable results much faster, within hours, whereas known methods required some days until the results were obtained.

Furthermore, a kit for a diagnostic method is provided, which can be used in diagnostic methods for detecting prion diseases, such as RT-QuIC and high-throughput applications of the method. This allows for routine-checks of foodstuffs and medical equipment, such as surgical instruments or blood products used for transfusion.

With the method of the present invention a second rPrP variant, also named $rPrP^{agg}$ in the following, can be isolated as a protein fraction from a known rPrP preparation. This second protein variant is eluted with high concentrations of imidazole after elution of the novel purified $rPrP^{ox}$ protein used for detection. This protein $rPrP^{agg}$ is characterized by its high purity and high affinity to self-aggregate. This protein can be used as positive control to assess test validity and to ensure that the correct experimental procedure has been followed. It has been surprisingly found, that this specific rPrP variant is not infectious and, therefore, can be safely used in a diagnostic test. Thus, the present invention also provides a safe protein that is superior to the infectious $PrP^{Sc}$ protein currently used as positive control, as shipping and handling of infectious material is expensive and requires specific safety measures. Thus, the method of the present invention not only provides a highly improved diagnostic agent - $rPrP^{ox}$ - and a diagnostic method with higher efficiency and cost-effectiveness, but in addition provides an improved protein to be used as positive control.

The isolation method of the present invention using specific selection steps, is rather selective and is not limited to isolate fractions of recombinant prion proteins, but can also be used to isolate fractions of other self-aggregating proteins, which cause other PMDs, such as Parkinson's, or Alzheimer's disease.

## Description of drawings

The invention is also explained with reference to the following figures.

Figures 1A-C show that detection of human sporadic CJD (sCJD) $PrP^{Sc}$ in cerebrospinal fluid (CSF) by RT-QuIC using conventionally purified rPrP showed both false-positive and false-negative results. CSF samples are obtained from four definite sCJD and four non-CJD patients. The readout is shown by Mars analysis software (BMG). Arrows represent the false-negatives, low activity and false-positive reactions. Reactions were tested at 37°C (A), 42°C (B) and 55°C (C), respectively. Increase of temperature not only increases protein activity, but also decreases the reliability of RT-QuIC reactions.

Figure 2 shows protein contamination in conventionally purified rPrP solution detected by liquid chromatography mass spectroscopy (LC-MS). The contaminating proteins are mainly bacterially expressed, including ATPase, GTPase and DNA polymerase, etc.. Two independent LC-MS tests indicated that the current purification method causes contamination, which may affect reliability of RT-QuIC. Both figures are direct output of the MASCOT software for LC-MS.

Figure 3 shows that rPrP of three independent peaks A, B, and C, is obtained by ICE. Imidazole concentrations of 100 mM, 300 mM, 500mM are used. The conventional elution profile showing a single peak obtained from IMAC using an imidazole gradient is shown as a small profile with gray background.

Figure 4 shows a comparison of the reliabilities of rPrPs in the different peaks obtained by the ICE as shown in Fig. 3 by using RT-QuIC. The rPrPs in peaks A, B and C were subjected to RT-QuIC for 90 hours at 55°C. The results show that rPrP in peak B is the reliable protein which can be applied in RT-QuIC for diagnostics. rPrP in peaks A and C should be excluded due to false negatives and false positives. rPrP in peak C can however be used as positive control in the test kit. The ascendant curve indicates the positive results. These data are obtained from Mars analysis software.

Figures 5A-C show the comparison of the performance of RT-QuIC for 90 h at 37°C and 55°C using rPrP in peak B as the substrate. RT-QuIC preparations were seeded with human sCJD, vCJD and non-CJD brain (e-7 represents the seed of $10^{-7}$ diluted brain) as shown in Fig. 5A. RT-QuIC preparations were seeded with human sCJD and non-CJD CSF (three cases for each, represented as 1, 2 and 3), as shown in Fig. 5B. RT-QuIC preparations were seeded with cattle classic BSE (C-BSE) and low-type BSE (L-BSE), as shown in Fig. 5C. The seeds used in the 37°C tests are identical to those in 55°C ones.

Figure 6 shows the detection of prions in CJD blood by using rPrP of peak B. The blood samples from both sCJD and vCJD patients (four per type) were precipitated and seeded into RT-QuIC for 55°C tests. Prions in vCJD blood can be detected on day 1 and those in sCJD blood are detectable on day 3 and 4, suggesting the application of peak B rPrP in high-throughput screening of blood samples.

Figure 7 shows that an oxidized methionine is found in peak B rPrP, but not in the rPrP of peaks A and C. This oxidized methionine is the methionine at residue 191 of SEQ ID NO:2. It corresponds to the methionine at residue 213 of the complete rPrP as defined in SEQ ID NO:4 (GenBank entry AAA60182.1) and encoded by the nucleotide sequence as defined in SEQ ID NO:3 (GenBank entry M13899.1). rPrPs were digested by trypsin and loaded on the C18 reversed phase column for LC-MS analysis. The oxidation of excess molecular weight of 64 Dalton is indicated by arrow in the diagram showing peak B. These figures are direct output of the MASCOT software for LC-MS.

Figure 8 shows that protein contamination is dramatically reduced in ICE prepared peaks B and C. Contaminating proteins from bacteria and the environment are mainly eluted in peak A. The solutions of peaks B and C are much clearer; therefore the purified rPrPs are much better suited for diagnostic methods like RT-QuIC or other prion tests.

Figure 9 shows the visible differences of the rPrP solutions before and after dialysis. The eluted rPrPs were sterilized by 0.22 μm filters first; then they were adjusted to 0.5 mg/ml with 10 KDa centrifugal filters before being loaded into 6 KDa dialysis tubing. Successive dialysis was done in 3 rounds (2 hours, 4 hours and 12 hours). Every dialysis was in 100 times volume of sterilized 10 mM $NaH_2PO_4$ (pH 5.6 at 4°C). Thus, the components in the eluted solution smaller than 6 KDa were diluted to $1\times10^6$ folds. After dialysis, the turbid solutions can be observed in peak A and C while peak B is still clean. For keeping similar volume of solution before and after dialysis, the loaded dialysis tubing was compressed by multiple clamps to reach the maximum pressure before being immersed in dialysis buffer.

Figure 10 shows that mixed solutions are turbid after dialysis. ICE eluted rPrP of peak B is pre-mixed with A and C, respectively. Both mixed solutions are clean before dialysis. However, the dialyzed solutions become muddy, indicating aggregates are formed in both mixes. Protein concentration is controlled to be 0.5 mg/ml.

Figure 11 shows that a concentration of 40 mM of imidazole does not result in elution of recombinant prion protein in a peak, but in a constant plateau at a low level.

Figure 12 shows that the elution of recombinant prion protein with 50 mM to 350 mM imidazole concentration harvested 6 peaks.

Figure 13 shows the elution profile of protein with 3 cascades of imidazole concentrations.

Figure 14 shows that the length of the chromatography column used in the selection method affects the required eluting duration to observe the peaks.

## Definitions

In this description and the claims, reference will be made to a number of terms which shall be defined to have the following meanings:

The terms "prion protein" and "PrP" are used as a general term for any type of prion protein and encompass recombinant PrP as well as naturally occurring PrP, mixtures of different variants of PrP as well as isolated forms. The term comprises human PrP and PrP from other animals like cattle, sheep, goat, and deer.

The term "rPrP" refers to recombinant PrP, i.e. PrP that has been obtained by genetic engineering methods and shall comprise pure proteins as well as a mixture of rPrP protein variants. This term refers to full-length proteins and fragments of PrP that are active in the present analytical methods.

The term "full-length protein" in the prion field refers to the mature human rPrP, which is a protein consisting of the amino acids at positions 23-230 as defined in SEQ ID NO:4, which corresponds to the GenBank entry AAA60182.1 for the human PrP. This GenBank entry is the protein, which is translated from the CDS of the PRNP gene, which is defined in SEQ ID NO:3 (GenBank entry M13899.1). However, the protein as defined in SEQ ID NO:4 is not the final form of cellular PrP. In the endoplasmic reticulum (ER), the part of the protein comprised of aa 1-22, which is a signal peptide of PrP, is cleaved first. It has been shown that this signal peptide affects the folding characteristics of the rPrP, and, therefore, should be absent from the mature rPrP of the present invention. Furthermore, part of the protein comprised of aa 231-253 is replaced by a glycosylphosphatidylinositol (GPI) anchor, which helps $PrP^{C}$ to locate on the cellular membrane. Therefore, only the protein consisting of amino acids at positions 23-230 is left and termed mature or full-length PrP. This protein is defined in SEQ ID NO:2. This full-length protein is encoded by the polynucleotide of SEQ ID NO:1, which corresponds to the nucleotides at positions 67-690 of the polynucleotide as defined in SEQ ID NO:3. The full-length rPrP is suitable to be the substrate for amplifying both human and cattle prions, and the yield of purified full-length rPrP is ideal. Furthermore, it has been shown that up to about 90 amino acids starting from the N-terminal methionine at position 1 of SEQ ID NO:4 can be digested by proteases without effecting the folding characteristics of the rPrP. Therefore it has been concluded that the essential part of the rPrP is consisting of the amino acids at positions 92-230 of SEQ ID NO:4. A protein of the present invention, therefore, is any full-length protein as defined in SEQ ID NO:2 as well as any protein that comprises the essential part but wherein some or all of the amino acids at positions 23-91 at the N-terminal part of the protein are missing and it comprises variants wherein some or all of the amino acids at positions 23-91 are varied by mutations, e.g. by deletions, insertion, and/or substitutions with other amino acids, e.g. with conservative substitutions. Conservative substitution tables are well known in the art, useful substitutions can be found by the skilled person by using such tables or by using routine experimentation. Examples for conservative substitutions are those wherein amino acids which originate from the same class are exchanged for one another, like amino acids having aliphatic side chains, positively or negatively charged side chains, aromatic groups in the side chains or amino acids, the side chains of which can enter into hydrogen bridges, e.g. side chains which have a hydroxyl function. Insertions, deletions and substitutions are possible as long as the folding characteristics of the rPrP are not essentially changed.

It has also been shown that the N-terminal region of PrP comprises an array of five almost identical octapeptide sequences, also termed octarepeats. This region is found at amino acid positions 51-91 of SEQ ID NO:4. One of these octarepeats can be consisting of amino acids Gln Pro His Gly Gly Gly Trp Gly (SEQ ID NO:5). These octarepeats have been shown to play a role in copper binding and homeostasis, as well as in protection from oxidative stress. Importantly, mutations that result in expansion of the octarepeats have been linked to familial Creutzfeldt-Jakob disease (CJD). Documented cases of familial CJD report insertions of 2-9 octapeptide sequences.

Therefore, variants of the rPrP of the present invention can comprise those peptides, which contain 2-9 octarepeats in their N-terminal region.

The term "$rPrP^{ox}$" refers to a recombinant PrP wherein methionine 213 is in oxidized form (wherein the numbering refers to the full length protein).

The term "$rPrP^{agg}$" refers to a new form of recombinant PrP that can be obtained with the method of the present invention as last fraction after elution of $rPrP^{ox}$ and differs from $rPrP^{ox}$ in that it self-aggregates without being infectious. It is assumed that this is a highly pure form of unoxidized rPrP that has self-aggregating properties.

The term "Prion disease" is used for all conditions, disorders or diseases that are caused by formation of prions, i.e. proteins that misfold and induce misfolding of other protein molecules. Examples for prion diseases are different types of Creutzfeldt-Jacob disease, BSE, FSE, TME, EUE, and CWD.

The terms "Imidazole cascade elution" and "ICE" refer to a method according to the present invention, wherein at least two imidazole fractions with different imidazole concentration are used for elution, wherein the at least two fractions have discrete concentrations.

The rPrP of the present invention can be used in detection methods for assaying the presence of $PrP^{Sc}$ in a sample. Useful detection techniques for prion diagnosis and drug screening include protein misfolding cyclic amplification (PMCA), amyloid seeding assay (ASA), scanning for intensely fluorescent targets (SIFT) assay, and RT-QuIC. The positive reaction in RT-QuIC is defined as showing an ascendant curve on the screening window of the RT-QuIC reaction within 96 hours. In particular, for RT-QuIC reaction seeded by cerebrospinal fluid (CSF), the positive reaction is defined as showing an ascendant curve within 48 hours. The flat line without ascendant curve in these time-spans is defined as a negative reaction.

By using the rPrP of the present invention in a detection method such as RT-QuIC prion diseases can be diagnosed and distinguished from other neurodegenerative disorders. Furthermore, samples, such as animal products, human blood, transplant tissues and surgery equipment, suspected to be contaminated with prions, can be tested for contamination. Moreover, therapeutic effects, including the tracking of prion progression after treating a subject with anti-prion therapy and possible side effects of treatments which may trigger spontaneous prion diseases while treating other diseases, can be estimated. Furthermore, an early diagnosis in a subject with a possible mutation of the PRNP gene, who may suffer from genetic prion diseases, and an early diagnosis for people who reach the age with high risk of getting prion diseases, can be carried out within the context of a routine physical examination of the subject.

## Detailed Description of the invention

The present invention provides a novel and inventive purification and isolation method, here also referred to as "Imidazole-cascade elution" (ICE), that allows to purify recombinant prion protein or other recombinant aggregating proteins and to obtain a highly pure and active protein that can be used as substrate in a detection method such as RT-QuIC. Furthermore, using the highly pure fraction obtained with this method, a sensitive diagnostic system for prion diseases is provided. Surprisingly, by using ICE, the inventors found a novel type of rPrP that has superior properties and is particularly useful in diagnostic methods.

The elution method of the present invention uses a chromatographic technique of protein purification based on the principle of immobilized metal ion affinity chromatography (IMAC). The bound recombinant proteins are eluted from the column using cascade concentrations of imidazole and at least two fractions can be obtained, one of which comprises the desired protein in highly pure form.

It was the surprising finding of the inventors that the rPrP used in the known testing methods was not as pure as assumed. Available rPrP, which had been purified in conventional form and had shown only one peak when checked for purity, was used by the inventors and they identified that the problem with known tests was the presence of protein variants that caused false-negative and false-positive results.

By analyzing the known protein with liquid chromatography mass spectroscopy (LC-MS) (see Fig. 2) existence of protein contamination in rPrP solutions was identified. Thus, it was found that following current chromatographic technique was inefficient to produce a reliable substrate for RT-QuIC. The inventors developed a new strategy to prepare highly pure rPrP with a much lower contamination and a higher activity for the RT-QuIC system.

It was found that conventionally prepared rPrP that was used in testing methods is a mixture of bacterial contaminants and of protein variants. This mixture can now be used as starting point for isolation of the protein of interest.

It was surprisingly found that by varying the concentration of imidazole used for elution by using cascade elution, an apparently homogenous protein source of self-aggregating protein can be differentiated into different fractions with different characteristics. The key innovation of imidazole cascade elution is to turn the common one-step (constant concentration of imidazole) and gradient elution (linear increased concentration of imidazole) into several separated cascades concentrations of imidazole for protein elution. These characteristics can be assessed, and the fraction containing the protein with the most beneficial characteristics for the intended use can be isolated with high purity and quality. This is shown with the example of the recombinant prion protein, which can be used for the diagnosis of TSEs. With ICE, not only protein with high activity, purity and reliability can be harvested, but the unspecific protein contamination and those proteins causing false-negative and false-positive results in RT-QuIC reactions can be excluded. In the present invention two, and an optional third, cascades were chosen, because it was found that rPrP on the chromatography column was composed of three subtypes with different chemical and biological features.

A further surprising finding of the present invention is the identification of the source of the problems of the conventional methods for diagnosis of prion diseases and the provision of the solution of said problems. These problems are the low reliability of the conventional diagnostic methods due to the prevalence of false negatives and false positives. A further problem was the low sensitivity and activity of the conventionally used detection agent leading to long detection times and the need for an increased amount of source material.

In general, purification via IMAC is a preferred technique when high yields of pure and active protein are required. Conventional IMAC is based on a high affinity binding of an immobilized metal ion by chelating a part of the target protein. Said part of the protein can be a histidine-rich sequence. Conventionally, target proteins are therefore tagged with an artificial histidine-rich sequence. The rPrP conventionally used in research is histidine-tagged as well (Colombo et al, PLoS one 2009; 4:e4296, [6]; Meli et al., PLoS one 2011, 6:e19093 [7]). This, however, obviously changes the nature of the protein, as its sequence is differing from the native sequence. As Histagged rPrP may not be useful in a method for detecting prion diseases, it was not advantageous to tag rPrP to be purified. In the method of the present invention, rPrP can be used with the native amino acid sequence as defined in SEQ ID NO:2 encoded by the genetic nucleotide sequence as defined in SEQ ID NO:1 or variants or fragments thereof as defined above and in the following. Variants of the rPrP of the present invention are in particular those peptides that comprise some or all amino acids 23-230 of SEQ ID NO:4, wherein amino acids 23-91 can be substituted by other amino acids, can be deleted or wherein in the part outside aa 92-230 insertions have been made, in other words wherein amino acids 92-230 of SEQ ID NO:4 are conserved. The substitutions preferably are conservative substitutions. Thus, the rPrP of the present invention comprises at least the amino acids at positions 92-230 of SEQ ID NO:4 and can comprise further amino acids of SEQ ID NO:4. The rPrP of the present invention can also consist of a peptide consisting of amino acids at positions 92-230 of SEQ ID NO:4. Furthermore, the rPrP of the present invention can comprise the peptide consisting of amino acids at positions 92-230 of SEQ ID NO:4, and an N-terminal region comprising from 2 to 9 octarepeats as defined in SEQ ID NO:5.

The rPrP used in the present invention can be obtained by recombinant expression in bacteria, followed by digestion, denaturation, and renaturation steps as are known to the person skilled in the art. As an example a method for obtaining the rPrP used in the methods of the present invention is described in brief, such methods are well-known in the art. The recombinant protein expressed by bacteria can be harvested by disrupting the bacteria cells by repeated freezing with liquid nitrogen followed by thawing. To harvest the recombinant protein said protein can be extracted from inclusion bodies by lysis, as is well-known in the art. In this lysis step a universal and efficient nuclease (e.g. Benzonase) can be used as well to digest total DNA and RNA to avoid the unspecific binding of nucleic acids to the solid support, such as a resin in a chromatography column. Nucleic acids comprise huge hydrophilic surfaces, which could cover the whole resin, and thus can block the binding sites (e.g., $Ni^{2+}$ on the resin) for the protein to be purified. Therefore, it is recommended to digest nucleic acids with Benzonase, or with a combination of multiple types of DNase and RNase, before denaturing proteins with guanidine hydrochloride. The following denaturation can be critical to give all rPrP in the inclusion body an identically denatured and non-structural starting state before refolding. The key of this step is to use 8 M or higher concentration of guanidine hydrochloride which is efficient for denaturation. In the preparation for rPrP used in the method of the present invention a concentration of 8 M guanidine hydrochloride in 50 mM Tris buffer (pH 8.5) was found preferable for dissolving the inclusion body and denaturing the recombinant PrP. Using a concentration of 6 M Guanidine hydrochloride was found to be insufficient. The following purification step can preferably be done at 2-8°C (normally 4°C) to keep protein activity. Therefore, urea, which is also a commonly used denaturant, is not useful, since low temperature (2-8°C) causes crystallization of dissolved urea. In the following the proteins are renaturated by allowing the proteins to refold. Refolding is carried out to obtain proteins having regained their native conformation/structure after denaturation. Refolding is carried out by decreasing denaturant concentration (e.g. guanidine hydrochloride) in the flowing aqueous phase. For refolding the rPrP of the present invention, the refolding should be done on the chromatography column. Furthermore, the refolding step preferably uses another denaturation buffer containing 6 M guanidine hydrochloride and sodium phosphates (for buffering pH value at 8.0), which are different from the solution of 8 M guanidine hydrochloride (pH 8.5) described above for the denaturation step. The reason is that > 6 M of Guanidine is not soluble at pH 8.0 with sodium phosphates at 4°C. Furthermore, the ingredients of the refolding buffer are the same as in the denaturation buffer except for guanidine hydrochloride, which is not present in the refolding buffer. Further details can be found in Example 7.

This protein, which is without an artificial His-tag, can nevertheless be purified using an IMAC method, as negative charged amino acids in the sequence provide for sufficient binding to the bivalent cations on the chromatographic support used in the method of the present invention. The cations bind to a native domain of the protein, namely the copper binding domain, which can bind bivalent cations such as $Zn^{2+}$, $Ca^{2+}$, $Cd^{2+}$, $Cu^{2+}$, $Co^{2+}$ and $Ni^{2+}$. Binding strength of PrP to the IMAC column depends on the cations used and follows the order $Cu^{2+} > Ni^{2+} > Zn^{2+} > Co^{2+}$. $Cu^{2+}$ ions, however, are not useful for the methods of the present invention, as the affinity of the copper ions is too high to efficiently elute the rPrP after binding to the metal ions. Preferably, the cations used to bind proteins are selected from $Zn^{2+}$, $Co^{2+}$, $Ca^{2+}$, $Cd^{2+}$, and $Ni^{2+}$, most preferably $Ni^{2+}$.

In a first step, the preparation comprising the recombinant prion protein obtained in conventional manner, preferably as outlined above, is subjected to a chromatographic separation step, such that the protein of interest can bind to a chromatography material comprising immobilized bivalent cations. Such chromatographic methods are known to the skilled person and chromatographic materials are available having metal ions immobilized on a solid support. Any such chromatographic material with immobilized bivalent cations can be used. Preferably, a chromatography column with immobilized $Ni^{2+}$ ions is used to bind recombinant proteins by utilizing the affinity of $Ni^{2+}$ to histidines in the protein sequence. One example for a material that is suitable for the chromatographic separation is a Ni-IDA resin as shown in the examples of the present application. Ni-IDA is a dry silica-based resin precharged with $Ni^{2+}$ ions. The chelating group of Ni-IDA is based on iminodiacetic acid (IDA), which enables strong and efficient binding of a target protein onto the IMAC matrix. The bivalent cations can also be immobilized on microbeads, microspheres, and planar support structures, etc. as is known to the skilled person.

Thus, the method of the present invention can be used to obtain an isolated, purified recombinant prion protein (rPrP) using immobilized metal ion affinity chromatography (IMAC). This method comprises immobilizing an rPrP preparation encoded by a nucleotide sequence comprising the nucleotide sequence as defined in SEQ ID NO:1 on a solid support coated with metal ions, such as $Zn^{2+}$, $Co^{2+}$, or $Ni^{2+}$.

The essential part of the method of the present invention is the elution of the protein of interest. As not only the protein of interest but further proteins are bound by the bivalent cations, the conditions of the elution are important to get rid of contaminants and to isolate the desirable protein. The proteins can be displaced from the column by using an agent competing for the binding to bivalent cations such as EDTA or imidazole. Conventionally, proteins are eluted with imidazole, which competitively binds to the metal ions, such as $Ni^{2+}$ ions. Thus, the recombinant prion protein can be eluted while washing the column with imidazole solution.

Currently, two elution methods are applied conventionally in IMAC for harvesting protein binding to a $Ni^{2+}$ column - isocratic or gradient elution. Isocratic elution using a constant concentration of imidazole is usually chosen in commercial kits. Gradient elution is reported for isolating rPrP for RT-QuIC [2,3]. It has been found that both approaches yield protein preparations that seem to be homogenous but by further analysis were found to comprise a mixture of protein variants and contaminants. In an analysis three peaks were found for a protein deemed to be pure: peak A comprising bacterial contaminants, peak B comprising the target protein $rPrP^{ox}$ and peak C comprising a self-aggregating protein variant $rPrP^{agg}$.

In contrast to the known elution techniques the present invention uses imidazole cascade elution - ICE, wherein cascades of increasing imidazole concentrations are used in sequence to elute fractions of the bound protein. At least two different eluent concentrations are applied. The appropriate concentrations of imidazole in the different eluents are critical and the desired fractions are only obtained in the concentration window outlined in detail below.

Generally, the eluent used for the present isolation method is an aqueous solution of imidazole that was found to displace the desired proteins most efficiently. The aqueous phase can be water, or a buffered solution, more preferred a phosphate buffered solution, phosphate buffered isotonic saline, having a pH in the range of 6.5 to 7.5, preferably about 7.0, or a TRIS-HCl buffer having a pH at about 8.0. The skilled person is aware of other aqueous buffer solutions useful for eluting proteins and those can be used as the aqueous phase in the method of the present invention, too. Imidazole is added to the aqueous phase in amounts as outlined in detail below. If necessary a minor amount of a tenside or other excipients can be added, as is known to the skilled person.

Imidazole cascade elution is the novel method of the present invention, therefore, at least two different eluents are necessary and at least two elution steps are carried out. In a first step contaminants are removed, thus the concentration of imidazole has to be selected such that the contaminants are eluted but not or only in a very minor amount of the desired prion protein. In a second step the target protein $rPrP^{ox}$ shall be eluted as exhaustively as possible but without eluting proteins of peak C. In an optional third elution step the protein $rPrP^{agg}$ of peak C can be eluted.

In the first elution step a low concentration of imidazole is used, that elutes contaminants but does not efficiently elute the recombinant prion protein. For the first step in the cascade the imidazole concentration should not be below 40 mM, as below 40 mM the contaminants are not eluted sufficiently. It was found that 40 mM imidazole elutes the protein to a constant plateau thus to cause a very low yield (see Fig. 11, and Example 1). Therefore, for the first elution step, the imidazole concentration shall be in the range of about 50 mM to about 150 mM. As shown above the lower threshold determines the minimal concentration necessary to elute a protein fraction in a peak. If the concentration is higher than 150 mM there is a risk that rPrP protein is eluted together with the contaminants, which lowers the yield of the desired fraction. The fraction eluted in this elution step comprises mainly bacterial and environmental protein contaminations, which are thought to be responsible for false negatives when used in the detection method, and for lowering the activity of the detecting agent. The fraction obtained in the first elution step comprising contaminants is to be discarded.

In a second elution step, an elution buffer with an increased imidazole concentration is used. This concentration is from about 150 mM to about 350 mM imidazole. It was shown that the fraction eluted with an aqueous solution, preferably a buffer solution like phosphate buffer, and an imidazole concentration in a range of about 150 mM to about 350 mM, preferably 175 mM to 300 mM contains the desired novel and inventive rPrP variant - $rPrP^{ox}$, which is a very active, safe and reliable detecting agent. This fraction can be used for a detection method or the $rPrP^{ox}$ can be recovered from the solution in well-known manner. The protein obtained is very stable and can be stored until it is used. Preferably, the protein is in aqueous solution and is frozen at -80°C until it is used.

After the second elution step remaining rPrP protein still bound to the chromatography matrix can be optionally eluted. It has been found that this remaining protein, in peak C, is a protein with valuable properties. Therefore, optionally a third elution step can be carried out to recover this protein, as well.

In the third elution step an elution buffer with a further increased imidazole concentration is used. A concentration of higher than 350 mM imidazole is used, preferably the imidazole concentration is from about 400 mM to about 500 mM imidazole. The upper limit is not critical, but a concentration of more than about 500 mM imidazole does not yield more protein and therefore is not useful. In the eluate of the third step a very pure rPrP variant is found - $rPrP^{agg}$, which is characterized by its high tendency to self-aggregate. It is assumed that it is this protein variant that causes false positives when the previously used rPrP preparation is applied. It can be beneficial to use this protein as positive control for the detection method of the present invention or generally for detection methods for detecting prion diseases to assess test validity and to ensure that the correct experimental procedure has been followed when carrying out the detection method. For this reason it is useful to separate the protein to be used as positive control from the fraction, which can be eluted with a buffer containing at least 350, preferably about 500 mM concentration of imidazole.

Further conditions of the elution method can be determined by a skilled person based on the following information, optionally by using routine experimentation.

The duration of elution steps can be determined by a person carrying out the method of the present invention in usual manner. The steps should have a sufficient length to elute the appropriate fraction. The time period depends on parameters like the size of the column, flow rate, temperature of the system, the chromatography system used, etc. For example, there are different sizes of containers for loading a chromatography column; for one size of container, different lengths of the column are available (see Fig. 14). Furthermore, the flow rate of the elution buffer can be adjusted by the operator using the provided function of the chromatography system, affecting the duration of elution. These issues influence the required duration for elution buffer to pass through the entire column. Since the monitor for detecting proteins is at the downstream position of the chromatography system, the time point of the appearance of the peak is variable. A person of skill in the art easily can determine the optimal elution time needed to obtain the desired fractions of protein.

Directly after elution rPrP can be transferred into a dialysis buffer. Dialysis steps are carried out as known to the person skilled in the art (see also Example 7). After dialysis, the concentration of rPrP is usually greater than 0.5 mg/ml. The concentration can then be adjusted to be 0.3 mg/ml to 0.5 mg/ml for long-term storage at -80°C or for direct use in a detection method. Further concentration steps, such as centrifugal filtration and lyophilization, are not necessary, and can be even detrimental. Centrifugal filtration causes loss of protein and increases the number of processing steps as well as the price of purification. Furthermore, the necessary low speed centrifugation for about 5 hours would expose the proteins to an enhanced risk of contamination. Lyophilization would also require additional steps like the exchange of dialysis buffer to $ddH_2O$ and the removal of water.

By using the isolation method of the present invention a novel and inventive recombinant protein is provided. The recombinant prion protein used in the ICE is encoded by the nucleotide sequence as defined in SEQ ID NO:1 or a fragment or variant thereof that encodes a fragment or variant as described above.. The translated amino acid sequence of the rPrP is defined in SEQ ID NO:2. An rPrP preparation as known and as used as starting material for the isolation method of the present invention can be obtained by bacterial expression, wherein the bacteria are transformed with a vector comprising an expression cassette for the rPrP. It is important that the sequence of the rPrP is not deviating from the native sequence of the $PrP^c$, because the folding mechanism is depending on the sequence and conformation of the protein. For this reason, no artificial tags are included in the protein sequence. Specifically, no His-tag is included. It is assumed that a deviating sequence might not or not in the same amount be induced to misfold by $PrP^{Sc}$ that is being tested.

The purified protein can be distinguished from the recombinant protein before purification. The purified rPrP of the invention has a distinctive oxidation pattern of the methionines in the amino acid sequence. Only one methionine is oxidized. This is the methionine at residue 213 of the full sequence, corresponding to residue 191 of SEQ ID NO:2 (..VVEQ**M**CITQYER..; aa 187-198, see also Fig. 7). This residue number of the oxidized methionine corresponds to the residue number 213 of the whole peptide sequence as defined in SEQ ID NO:4 (see also GenBank entry AAA60182.1: http://www.ncbi.nlm.nih.gov/protein/190468).

As shown above and below, the peptide sequence as defined in SEQ ID NO:4 includes a signal peptide at amino acid positions 1-22, and a peptide sequence at amino acid positions 231-253, which are cleaved from the peptide to obtain the full-length PrP as defined in SEQ ID NO:2. This PrP consists of 208 amino acids. The oxidized methionine is at residue 191, which corresponds to residue 213 of the complete peptide as defined in SEQD ID NO:4.

Whole peptide sequence (SEQ ID NO:4) consisting of the full-length rPrP as defined in SEQ ID NO:2 (shown underlined) and the peptide sequences (shown in italics), which are cleaved. The oxidized methionine is shown in bold:

*MANLGCWMLVLFVATWSDLGLC*KKRPKPGGWNTGGSRYPGQGSPGGNRYPPQG
GGGWGQPHGGGWGQPHGGGWGQPHGGGWGQPHGGGWGQGGGTHSQWNK
PSKPKTNMKHMAGAAAAGAVVGGLGGYMLGSAMSRPIIHFGSDYEDRYYRENMH
RYPNQVYYRPMDEYSNQNNFVHDCVNITIKQHTVTTTTKGENFTETDVKMMERVVE
Q**M**CITQYERESQAYYQRGS*SMVLFSSPPVILLISFLIFLIVG*

Oxidation on methionines has been reported to affect the conformational stabilization of the prion protein (Wolschner et al., Proc Natl Acad Sci U S A.2009; 106:7756-7761 [8]). Met213, corresponding to Met191 of the novel and inventive rPrP, which is on $\alpha$-helix 3 of PrP, is thought to be a crucial amino acid to control the conformational stability. It has been hypothesized that oxidation of this methionine may function as a switch to trigger the pathogenic conversion of PrP [6]. The research as reported in [6,8] was done in computer simulations. As, however, targeted oxidation of a specific methionine is not possible *in vitro,* it was previously not possible to provide a rPrP with the oxidation pattern as claimed. With the novel separation method of the present invention it was possible to provide said $rPrP^{ox}$ with the claimed oxidation pattern. Without being bound by theory it is concluded that it is the oxidation of Met191/213 that accounts for the activity difference between $rPrP^{ox}$, the protein in peak B, of the present invention and an rPrP preparation known in the prior art or rPrPs in other peaks and thus provides the reliability necessary for the detection method.

Furthermore, it was found that not only human rPrP, but also rPrP of other animals has different types on the column while being purified with IMAC. For example, it was found that full-length (mature) mouse rPrP (aa 23-231) purified with the methods of the present invention was composed of different subtypes of rPrP with independent oxidation and converting activity features.

With the ICE a further recombinant protein can be provided, which can be separated from the rPrP mixture in a further elution step subsequently to the elution of $rPrP^{ox}$. The further elution step uses a high concentration of imidazole, which should be at least 450 mM, preferably at least 500 mM. The rPrP eluted with this high concentration of imidazole is characterized by its very high purity, and its high affinity to self-aggregate. This protein, $rPrP^{agg}$, differs from $rPrP^{ox}$ by the missing oxidation at methionine 213. This protein can be used in the test as a positive control. This is superior to the infectious $PrP^{Sc}$ protein currently used as positive control, as shipping and handling of infectious material is expensive and requires specific safety measures. These were not necessary if the protein of the present invention were used as positive control.

Furthermore, a kit for isolating $rPrP^{ox}$ is provided comprising the following reagents:

a rPrP preparation; a first eluent comprising 50 mM to up to 150 mM imidazole in aqueous solution; a second eluent comprising from about 150 mM to up to about 350 mM imidazole in aqueous solution; optionally a third eluent comprising from about 350 mM to about 500 mM imidazole. Additionally, an inclusion body extracting buffer (e.g. 150 mM NaCl, 50 mM Tris, 1% Triton X-100, 1% sodium deoxycholate, 0.5% Sarkosyl, 2 mM $Mg^{2+}$, 2.5 U/ml of benzonase and 1,000 kU/ml lysozyme, pH 7.4); a first denaturation buffer 1 for denaturing rPrP in inclusion bodies (e.g. 8 M Guanidine hydrochloride, 50 mM Tris, pH 8.5); a second denaturation buffer 2 for refolding (e.g. 6 M Guanidine hydrochloride, 95 mM $Na_2HPO_4$, 5 mM $Na_2HPO_4$, pH 8.0); a refolding buffer (e.g. 95 mM $Na_2HPO_4$, 5 mM $Na_2HPO_4$, pH 8.0); an elution buffer (e.g. 5 mM $Na_2HPO_4$, 95 mM $Na_2HPO_4$, 500 mM imidazole, pH 5.8); and a dialysis buffer (e.g. 0.5 mM $Na_2HPO_4$, 9.5 mM $Na_2HPO_4$, pH 5.8) can be included.

The new proteins $rPrP^{ox}$ and $rPrP^{agg}$ can be used in a detection method such as RT-QuIC. Thus, a further object of the present invention is a method for detecting a prion disease in a sample using a RT-QuIC test. For this purpose, a kit for detecting $PrP^{sc}$ in a sample using the $rPrP^{ox}$ of the present invention is provided comprising the following reagents:

5 × RT-QuIC reaction buffer (e.g. 2 M NaCl, 25 mM $Na_2HPO_4$, 25 mM $Na_2HPO_4$, 5 mM EDTA); 1 mM Thioflavin T; the human $rPrP^{ox}$ of the present invention; and, optionally the $rPrP^{agg}$ of the present invention for use as a positive control.

The buffer specifications for the isolation/purification and detection kits are intended to be exemplary. The person skilled in the art is capable of adjusting or employing buffers with differing specifications, which nevertheless are encompassed by the scope of the present invention.

**Examples**

Preferred embodiments of the invention are outlined in the following examples which should not be interpreted as restricting the scope or spirit of the invention.

**Example 1: Determination of elution profile**

The appropriate concentration of imidazole for elution was tested since low concentrations of imidazole do not efficiently elute the recombinant prion protein. It was shown that e.g., 40 mM imidazole elutes the protein to a constant plateau and, thus, causes a very low yield (see Fig. 11).

In order to find a better starting concentration of imidazole for ICE, 7 concentration cascades (50 mM to 350 mM) were used to elute protein from the column (see Fig. 12). Each concentration was performed 60 min. A total of 6 peaks were observed, the first peak (peak 1) appeared while using 50 mM imidazole. Then almost every concentration cascade of imidazole generated one peak (peak 2 to 6) but 250 mM. After dialysis the eluted proteins in these peaks, protein concentrations were adjusted to 0.5 mg/ml followed by measuring their turbidities with 340 nm (A340) absorption (see Table 1). The result showed that proteins in peak 1, 2 and 6 had higher turbidities than those in peak 3 to 5, indicating that peak 1, 2 and 6 contain proteins which are easily forming aggregates. Protein aggregates are harmful to either production or using as substrate in diagnosis, since (i) aggregated protein having large size can hardly pass through the next 0.22 μm sterilizing filter, (ii) spontaneously formed protein aggregates cause false-positivity in diagnosis. Therefore, peak 1, 2 and 6 should be discarded from the yield. It can conclude that, imidazole concentrations range from 150 mM to 300 mM can elute appropriate protein for RT-QuIC based prion diagnosis; lower or higher concentrations elute proteins forming spontaneous aggregates.

**Table 1:** The turbidity (A340) of protein solutions in peak 1 to 6

| absorption | peak 1 | peak 2 | peak 3 | peak 4 | peak 5 | peak 6 |
|---|---|---|---|---|---|---|
| A280 | 1.28 ±0.019 | 1.36 ±0.072 | 1.31 ±0.068 | 1.33 ±0.029 | 1.35 ±0.056 | 1.27 ±0.17 |
| A340 | 0.96 ±0.026 | 1.12 ±0.17 | 0.004 ±0.001 | 0.006 ±0.003 | 0.007 ±0.001 | 0.99 ±0.13 |

A280 indicates protein concentration. The protein is 0.5 mg/ml when A280 is 1.3.

A340 indicates turbidity of protein solution.

To optimize the elution procedure, the 7 cascades of imidazole concentrations were decreased to 3. Concentrations of 100 mM, 300 mM and 500 mM imidazole for elution were chosen (see Fig. 3 and Fig. 13). Thus, the previous peak 1 and 2 were eluted with peak A, peak 3 to 5 were in peak B, and peak 6 and the remaining proteins were in peak C. This elution profile is unexpected and quite different from known single peaks obtained from IMAC (represented as a small profile with gray background in Fig. 3), since the base of IMAC is to purify homogenized protein. However, these elution peaks indicate that rPrP is separated into three fractions, suggesting that the protonization level of the protein given by imidazole in each peak is different from one to another. Therefore, it was concluded that rPrP in three peaks were chemically different.

**Example 2: Using ICE-prepared rPrP in RT-QuIC to detect prions in human CJD and cattle BSE**

To estimate the reliability of rPrPs prepared by ICE, the proteins were subjected to RT-QuIC reactions including non-seeded and sCJD-seeded preparations (see Fig. 4). Each reaction had six repeats and was performed at 55°C for 90 hours. For non-seeded RT-QuIC, proteins contained in peaks A, B, and in a mixture of both peaks A and B (A+B mix) were observed to be stable, showing flat signals. However, the proteins contained in both peaks C and C+B mix cause false-positives. For sCJD-seeded ($10^{-10}$ diluted sCJD MM1 brain), peak A showed a false-negative result while other preparations were active. These results indicate that peak B contains the most reliable rPrP for RT-QuIC, and peak A and C cause false-negativity and false-positivity, respectively, should be excluded from harvested rPrP to be used as detection agent in a diagnostic method.

**Example 3: Influence of reaction temperature**

Two temperature points were chosen (37°C and 55°C) to further assess the activity of rPrP in peak B (see Fig. 5). RT-QuIC preparations using peak B rPrP were seeded with serially diluted human sCJD and variant CJD (vCJD) brain, human sCJD CSF, classic cattle BSE (C-BSE) and low type cattle BSE (L-BSE) brain, respectively. These results show that rPrP of peak B can work for detecting sCJD, vCJD, C-BSE and L-BSE prions without causing false-positives in either species, indicating the suitability for RT-QuIC based prion diagnosis. Meanwhile, both 55°C and 37°C can work for RT-QuIC, indicating rPrP in peak B has a wide range of working temperature with high stability, suggesting the peak B rPrP can be applied not only to prion diagnosis, but also to investigate prion propagation. Moreover, RT-QuIC using peak B rPrP has higher efficiency at 55°C than 37°C, which can dramatically shorten diagnosis duration, i.e. half days shorter for detecting prions in human CSF and L-BSE. This ability of reliably reacting at high temperature gives RT-QuIC the highest efficiency compared to other studies (Peden et al., J. Gen. Virol 2012; 93:438-449 [9]; McGuire et al., Prion 2011;5:18 [10]).

Since the stability and higher efficiency of RT-QuIC at 55°C had been confirmed, it was concluded that the peak B of rPrP could be applied to detect prions in human CJD blood. To address this, whole blood extract was spiked into RT-QuIC preparations and was subjected to 55°C RT-QuIC for 90 h (see Fig. 6). Four reactions seeded by vCJD blood samples showed positive results on day 1. Surprisingly, the sCJD blood-seeded RT-QuIC also showed positive results on day 3 and day 4. Detection of $PrP^{Sc}$ in sCJD blood samples has never been reported before. All eight non-CJD blood samples were negative after 90 h. These results indicate that the rPrP contained in peak B with high stability at high temperature is efficient to detect prions in human sCJD and vCJD samples, suggesting that this rPrP has a very high sensitivity in RT-QuIC for high-throughput screening of blood samples.

**Example 4: Analysis of protein and protein solution**

To characterize the chemical properties of ICE-prepared rPrP, rPrP in peaks A, B and C was subjected to LC-MS. Interestingly, it was found that the methionine at amino acid position 191 (corresponding to the methionine at amino acid position 213 of the peptide as defined in SEQ ID NO:4) but no other methionine of the amino acid sequence of peak B rPrP had been oxidized. No oxidation of any methionine could be found in the amino acid sequences of the rPrP contained in either peak A or peak C. Oxidation on methionines has been reported to affect the conformational stabilization of the prion protein [8]. Met213, corresponding to Met191 of the novel and inventive rPrP, which is on $\alpha$-helix 3 of PrP, is thought to be a crucial amino acid to control the conformational stability. It has been hypothesized that oxidation of this methionine may function as a switch to trigger the pathogenic conversion of PrP [6]. Hence, it was concluded that oxidation of Met191/213 accounts for the activity difference between peak B rPrP to rPrPs in other peaks. Active oxidation of a specific methionine is however not possible in vitro.

**Example 5: Protein contamination**

Although IMAC is thought to be specific for purifying protein, the protein contamination from cultured bacterial and environment can hardly be avoided. As shown in Fig. 2, some unexpected proteins which are not rPrP are always detected by LC-MS. By testing ICE-prepared rPrP, it was surprisingly found that the main protein contamination was only shown in peak A but not in B and C (see Fig. 8). These data suggest that ICE can produce much purer rPrP not only for RT-QuIC, but also for other prion studies with biochemical and biophysical methods requiring low background and weak interference coming from other proteins. It also suggested that ICE is an ideal method to increase purity while purifying other proteins from $Ni^{2+}$ resin.

**Example 6: ICE-prepared protein of peak B has a low risk to form self-aggregates**

The last step of protein purification is usually dialysis or centrifugal filtration for buffer-exchanging and harvesting large amounts of protein. It is quite common that proteins form visible aggregates, especially while purifying rPrP. However, these aggregates can cause substantial loss of protein if 0.22 $\mu$m filtrating sterilization is required. For RT-QuIC, 0.22 $\mu$m filtration is essential since measuring thioflavin T fluorescence requires a clean solution for better transmittance. Meanwhile, sterilization can avoid the growth of microorganisms during the warm incubation. Moreover, these rPrP aggregates should be removed since they may induce spontaneous conversion to cause false-positive results. The fractions containing the peaks obtained by ICE were dialyzed and it was found that although all three solutions were clean before dialysis, peaks A and peak C, but not peak B, showed visible turbidity after dialysis, indicating that rPrP in peaks A and peak C can form aggregates automatically, which are different from transparent rPrP solution in peak B (see Fig. 9). After filtrating the dialyzed solutions with a 0.22 $\mu$m filter, substantial losses of rPrP in peaks A and C were determined, but not in peak B (see Table 2). Similarly, pre-mixing the peaks before dialysis led to muddy solutions (see Fig. 10), which induced protein loss after filtration (see Table 3). These data indicate that, (1) rPrP in peaks A and C can form aggregates, they may also trigger aggregation of peak B rPrP; (2) these aggregates reduce the yield of rPrP for RT-QuIC; (3) self-aggregation of peak C rPrP may be the reason for causing false-positives in RT-QuIC; (4) ICE can help to exclude rPrP, which forms aggregates, and to increase the yield of protein; this also suggests a commercial value of producing proteins with ICE.

**Table 2:** The absorptions of dialyzed rPrP in peaks at 280 nm (for protein concentration) and 340 nm (for solution turbidity)

| absorption | rPrP in peak | | | 0.22 $\mu$m filtrated rPrP in peak | | |
|---|---|---|---|---|---|---|
| | peak A | peak B | peak C | peak A | peak B | peak C |
| A280 | 1.34 $\pm$0.047 | 1.33 $\pm$0.065 | 1.29 $\pm$0.150 | 0.53 $\pm$0.015 (40%) | 1.19 $\pm$0.028 (89%) | 0.41 $\pm$0.084 (32%) |
| A340 | 1.10 $\pm$0.025 | 0.094 $\pm$0.021 | 1.043 $\pm$0.060 | 0.009 $\pm$0.003 | 0.003 $\pm$0.002 | 0.004 $\pm$0.001 |

Dialyzed rPrPs were kept at 0.5 mg/ml to compare their turbidities. The rPrPs in peaks A and C show more than 10 times higher turbidity than that in peak B. Although the turbidity of these solutions is decreased after 0.22 $\mu$m filtration, the yields in peaks A and C are quite low (40% and 32%, respectively), comparing to 89% yield of peak B, suggesting most of rPrP aggregates in A and C are excluded by filtration. Absorption is shown with mean$\pm$SD from three independent batches of rPrP.

**Table 3:** The absorptions of dialyzed peak mix

| absorption | rPrP in peak | | 0.22 $\mu$m filtrated rPrP in peak | |
|---|---|---|---|---|
| | peak A + peak B | peak C + peak B | peak A + peak B | peak C + peak B |
| A280 | 1.28 $\pm$0.061 | 1.33 $\pm$0.029 | 0.68 $\pm$0.042 (53%) | 0.60 $\pm$0.035 (45%) |

(continued)

| absorption | rPrP in peak | | 0.22 μm filtrated rPrP in peak | |
|---|---|---|---|---|
| | peak A + peak B | peak C + peak B | peak A + peak B | peak C + peak B |
| A340 | 1.01 ±0.037 | 1.03 ±0.091 | 0.006 ±0.003 | 0.007 ±0.002 |

Both mixes of peaks A+B and peaks C+B cause high turbidity, suggesting rPrPs in peak A and C assemble rPrP of peak B thus causing aggregation. The yields are only 53% and 45%, respectively, after 0.22 μm filtration, indicating the loss of half the rPrP due to aggregation. The ratio of rPrP amount of peak B: peak A or C is 10: 1. Absorption is shown with mean±SD from three independent batches of rPrP.

**Example 7: Protocol for Imidazole cascade elution (ICE)**

**Gene sequence of recombinant prion protein (SEQ ID NO: 1)**

aagaagcgcccgaagcctggaggatggaacactgggggcagccgatacccggggcagggcagccctggaggc
aaccgctacccacctcagggcggtggtggctgggggcagcctcatggtggtggctgggggcagcctcatggtggtg
gctgggggcagccccatggtggtggctggggacagcctcatggtggtggctggggtcaaggaggtggcacccaca
gtcagtggaacaagccgagtaagccaaaaaccaacatgaagcacatggctggtgctgcagcagctggggcagtg
gtgggggccttggcggctacatgctgggaagtgccatgagcaggcccatcatacatttcggcagtgactatgagga
ccgttactatcgtgaaaacatgcaccgttaccccaaccaagtgtactacaggcccatggatgagtacagcaaccaga
acaactttgtgcacgactgcgtcaatatcacaatcaagcagcacacggtcaccacaaccaccaagggggagaactt
caccgagaccgacgttaagatgatggagcgcgtggttgagcagatgtgtatcacccagtacgagagggaatctcag
gcctattaccagagaggatcg

**Amino acid sequence of recombinant prion protein (SEQ ID NO: 2)**

kkrpkpggwntggsrypgqgspggnryppqggggwgqphgggwgqphgggwgqphgggwgqphgggwg
qgggthsqwnkpskpktnmkhmagaaaagavvgglggymlgsamsrpiihfgsdyedryyrenmhrypnqvy
yrpmdeysnqnnfvhdcvnitikqhtvttttkgenftetdvkmmervveqmcitqyeresqayyqrgs

**rPrP expression**

BL21 (DE3) E.coli. and pET41 a system were used to express rPrP. Bacteria were cultured in TB medium at 37°C with 200 to 220 rpm shaking till OD reached 1.5 to 2.0. Next, 1 mM of IPTG (final concentration) were added into medium for induction at 37°C for 10 to 16 h with 220 to 300 rpm shaking. The cell pellet was harvested by 10,000 g centrifugation at 4°C for 10 min and the wet weight (gram) was measured. Then the cells were frozen with liquid nitrogen followed by thawing at 25°C. This step was repeated twice to thoroughly break the cell.

**Inclusion body extraction**

For each one gram of cell (wet), 10 ml of lysis buffer (pH 7.4) was added for dissolving. The lysis buffer contained 150 mM NaCl, 50 mM Tris, 1% Triton X-100, 1% sodium deoxycholate, 0.5% Sarkosyl, 2 mM $Mg^{2+}$, 2.5 U/ml of Benzonase and 1,000 kU/ml lysozyme followed by shaking at 25 to 30°C for 1 h to dissolve the bacteria. Thereafter, this solution was centrifuged with 16,000 g at 4°C for 20 min to harvest pellet. The pellet was resuspended by 10 ml of lysis buffer to repeat the dissolving and centrifugation twice. Then the pellet was firstly resuspended by 10 ml of lysis buffer followed by adding 40 ml of $ddH_2O$ and mixing. This solution was precipitated by 10,000 g centrifugation at 4°C for 10 min. The harvested pellet was resuspended by 0.1 $\times$ lysis buffer followed by 16,000 g centrifugation at 4°C for 20 min. The final inclusion body pellet (from 1 g of wet cell) was denatured with 10 ml of denaturation buffer (50 mM Tris, 8 M Guanidine hydrochloride, pH 8.5) at 25°C for 1 to 3 h. The denatured protein solution was centrifuged at 16,000 g at 4°C for 30 min to remove the debris.

**Protein purification**

$Ni^{2+}$-IDA column (resin) was chosen for protein purification. Following buffers were required for this purification:

Regeneration buffer: 6 M Guanidine hydrochloride, 200 mM Acetic acid, pH 4.0

Denaturation buffer: 6 M Guanidine hydrochloride, 95 mM $Na_2HPO_4$, 5 mM $NaH_2PO_4$, pH 8.0

Refolding buffer: 95 mM $Na_2HPO_4$, 5 mM $NaH_2PO_4$, pH 8.0

Elution buffer: 5 mM $Na_2HPO_4$, 95 mM $NaH_2PO_4$, 500 mM imidazole, pH 5.8

Dialysis buffer: 0.5 mM $Na_2HPO_4$, 9.5 mM $NaH_2PO_4$, pH 5.8

These buffers were sterilized by 0.22 $\mu$m filtration and chilled at 4°C till use.

IDA resin was firstly regenerated by 2 volumes of regeneration buffer followed by washing with 3 volumes of denaturation buffer at 25°C (room temperature). Thereafter, denatured protein solution was mixed with treated IDA resin for protein binding at 25°C for 1 h with gently shaking. After removing the solution, IDA resin was loaded onto the AKTAprime chromatography system for refolding and harvesting the recombinant prion protein at 4°C (see below).

A gradient of buffer exchange was used for refolding step. The denaturation buffer was set from 100% to 0%, and refolding buffer was from 0% to 100%. Buffer exchange went through the resin at 4°C for 15 h with flow rate 0.3 ml/min. Then the resin was washed by 10 volumes of refolding buffer at 4°C for 1 h with flow rate 0.5 ml/min. The imidazole cascade elution was done at 4°C with constant flow rate 2 ml/min. First, the elution buffer was injected from 0% to 20% (containing 100 mM imidazole), and the refolding buffer decreased from 100% to 80%. Buffers ran through the IDA column by keeping these ratios till reaching the end of the first eluting peak (peak A). Then the elution buffer was adjusted to 60% (300 mM imidazole) and refolding buffer was 40%. This step was finished when the second peak (peak B) was at the end. Then the elution buffer was adjusted to 100% for eluting the rest protein (peak C).

The eluted protein solutions (peak A, B and C) were loaded into dialysis tubing (MWCO 6000 to 8000) individually after 0.22 $\mu$m filtration. The loaded tubing was immersed in 100 volumes of pre-chilled dialysis buffer for three times of dialysis. The durations of dialysis were 2 h, 4 h and 15 h, respectively. The dialyzed proteins were harvested on ice after 0.22 $\mu$m filtration. The protein solutions were measured with 280 nm (A280) absorption followed by calculating the protein concentrations with the formula below:

$$A280 = 2.67 \times \text{protein conc. (mg/ml)} \times \text{cuvette length (1 cm)}$$

At last, the protein solutions were divided into aliquots and frozen with liquid nitrogen followed by transferring to -80°C for long-term storage.

**References**

[1] Atarashi R, Wilham JM, Christensen L et al. Simplified ultrasensitive detection of recombinant PrP conversion with shaking. Nat Methods 2008;5:211-212.

[2] Wilham JM, Orru CD, Bessen RA, et al. Rapid end-point quantitation of prion seeding activity with sensitivity comparable to bioassays. PLoS 2010;6(12).

[3] Atarashi R, Satoh K, Sano K et al. Ultrasensitive human prion detection in cerebrospinal fluid by real-time quaking induced conversion. Nature Medicine 2011; 17(1).

[4] Atarashi R, Sano K, Satoh K et al. Real-time quaking-induced conversion: a highly sensitive assay for prion detection. Prion 2011; 5:150-153.

[5] Orru CD, Wilham JM, Hughson AG, et al. Human variant Creutzfeldt-Jakob disease and sheep scrapie PrP(res) detection using seeded conversion of recombinant prion protein. Protein Eng Des Sel. 2009; 22: 515-521.

[6] Colombo G, Meli M, Morra G, Gabizon R, Gasset M. Methionine sulfoxides on prion protein Helix-3 switch on the alpha-fold destabilization required for conversion. PLoS one 2009; 4:e4296

[7] Meli M, Gasset M, Colombo G. Dynamic Diagnosis of Familial Prion Disease Supports the $\beta$2-$\alpha$2 Loop as a Universal Interference Target. PLoS one 2011, 6:e19093

[8] Wolschner C, Giese A, Kretzschmar HA, et al. Design of anti- and pro-aggregation variants to assess the effects of methionine oxidation in human prion protein. Proc Natl Acad Sci U S A.2009;106:7756-7761.

[9] Peden AH, McGuire LI, Appleford NE et al. Sensitive and specific detection of sporadic Creutzfeldt-Jakob disease brain prion protein using real-time quaking-induced conversion. J. Gen. Virol 2012; 93:438-449.

[10] McGuire L, Peden A, Appleford N et al. Prion seeding activity in cerebrospinal fluid from sporadic Creutzfeldt-Jakob disease patients using real-time QuIC analysis: a potential new diagnostic test? Prion 2011;5:18.

## Claims

**1.** A method of obtaining an isolated, purified recombinant prion protein (rPrP$^{ox}$) using immobilized metal ion affinity chromatography (IMAC), wherein the method comprises

a) immobilizing a prion protein preparation on a solid support carrying bivalent cations,
b) eluting a first fraction of proteins from the solid support with an aqueous phase containing from about 50 mM to up to about 150 mM imidazole,
c) eluting a second fraction of rPrP$^{ox}$ from the solid support with a buffer containing from 150 mM to about 350 mM imidazole,
d) collecting the second fraction, containing the isolated purified rPrP$^{ox}$.

2. The method of claim 1, wherein the prion protein preparation comprises rPrP or a fragment or variant thereof.

3. The method of claim 2, wherein the rPrP comprises a peptide sequence as defined by the amino acids at positions 92-230 of SEQ ID NO:4, or wherein the rPrP comprises at least amino acids at positions 92-230 of SEQ ID NO:4 wherein the rPrP consists of a peptide sequence as defined by the amino acids at positions 92-230 of SEQ ID NO:4, or wherein the rPrP consists of a peptide sequence as defined in SEQ ID NO:2, or wherein the rPrP comprises a peptide sequence as defined by the amino acids at positions 92-230 of SEQ ID NO:4 and an N-terminal region comprising a fragment of the peptide sequence as defined by the amino acids at positions 23-92 of SEQ ID NO:4, wherein the fragment can comprise mutations such as deletions, insertion, and/or substitutions, such as conservative substitutions, or wherein the rPrP comprises a peptide sequence as defined by the amino acids at positions 92-230 of SEQ ID NO:4 and a N-terminal region, wherein the N-terminal region comprises from 2 to 9 octarepeats as defined by SEQ ID NO:5; or wherein the rPrP is an rPrP variant comprising amino acids 92-230 of SEQ ID NO:4 and additionally at least part of the sequence of amino acids 23-91, wherein some of amino acids 23-91 are substituted by other amino acids, are deleted or wherein insertions have been made.

4. The method of any one of the preceding claims, wherein the cations are selected from $Zn^{2+}$, $Co^{2+}$, or $Ni^{2+}$, preferably wherein the cations are $Ni^{2+}$.

5. Method of any one of the preceding claims, wherein the solid support is selected from the group consisting of a resin, microbeads, microspheres, and planar supports.

6. Method of any one of the preceding claims, wherein the solid support is a Ni-IDA resin.

7. Isolated purified rPrP obtained with the method of any one of claims 1 to 6.

8. Isolated purified rPrP comprising or consisting of an amino acid sequence as defined in claim 2 or 3, wherein the only oxidized methionine in the sequence is the methionine corresponding to the methionine at position 191/213 of SEQ ID NO:2/4, and wherein the isolated purified rPrP does not comprise an artificial his-tag.

9. Use of the isolated rPrP of claims 7 or 8 in a method for detecting scrapie prion protein $PrP^{Sc}$ in a sample, wherein the detection of aggregates of the rPrP indicates the presence of $PrP^{Sc}$ in the sample.

10. Use of claim 9, wherein the detection method is selected from the group comprising protein misfolding cyclic amplification (PMCA), amyloid seeding assay (ASA), scanning for intensely fluorescent targets (SIFT) assay, and real-time quaking-induced conversion (RT-QuIC), preferably wherein the detection method is RT-QuIC.

11. Use according to claim 10, wherein the detection method, preferably RT-QuIC, is carried out at a temperature from about 37°C to about 55°C.

12. Method according to claim 1, further comprising a further elution step e) after step c) of claim 1, wherein a third fraction of $rPrP^{agg}$ is eluted from the solid support with a buffer containing at least about 350 mM and up to 500 mM imidazole, and a further step f) of collecting said third fraction.

13. Use of $rPrP^{agg}$, contained in the third fraction obtained by the method of claim 12, as a positive control in a method for detecting scrapie prion protein $PrP^{Sc}$ in a sample using a detection method selected from the group comprising PMCA, ASA, SIFT assay, and RT-QuIC, preferably RT-QuIC.

14. Use of any one of claims 9 to 10, or 13, wherein the sample is obtained from foodstuff, a liquid such as blood of a human, or an animal such as cow, sheep, deer, cat etc., medical equipment such as surgical instruments.

15. Kit for isolating $rPrP^{ox}$ comprising the following reagents: a rPrP preparation; a first eluent comprising 50 mM to up to 150 mM imidazole in aqueous solution; a second eluent comprising from about 150 mM to up to about 350 mM imidazole in aqueous solution; optionally a third eluent comprising from about 350 mM to about 500 mM imidazole; an inclusion body extracting buffer; a first denaturation buffer 1 for denaturing rPrP in inclusion bodies; a second denaturation buffer 2 for refolding; a refolding buffer; an elution buffer; and a dialysis buffer.

16. Kit for detecting prion diseases comprising: $rPrP^{ox}$ as obtained with a method of one of claims 1 to 5 or as defined in claim 7 or 8 as detecting agent; a reaction buffer; 1 mM Thioflavin T; and, optionally, the $rPrP^{agg}$ for use as a positive control.

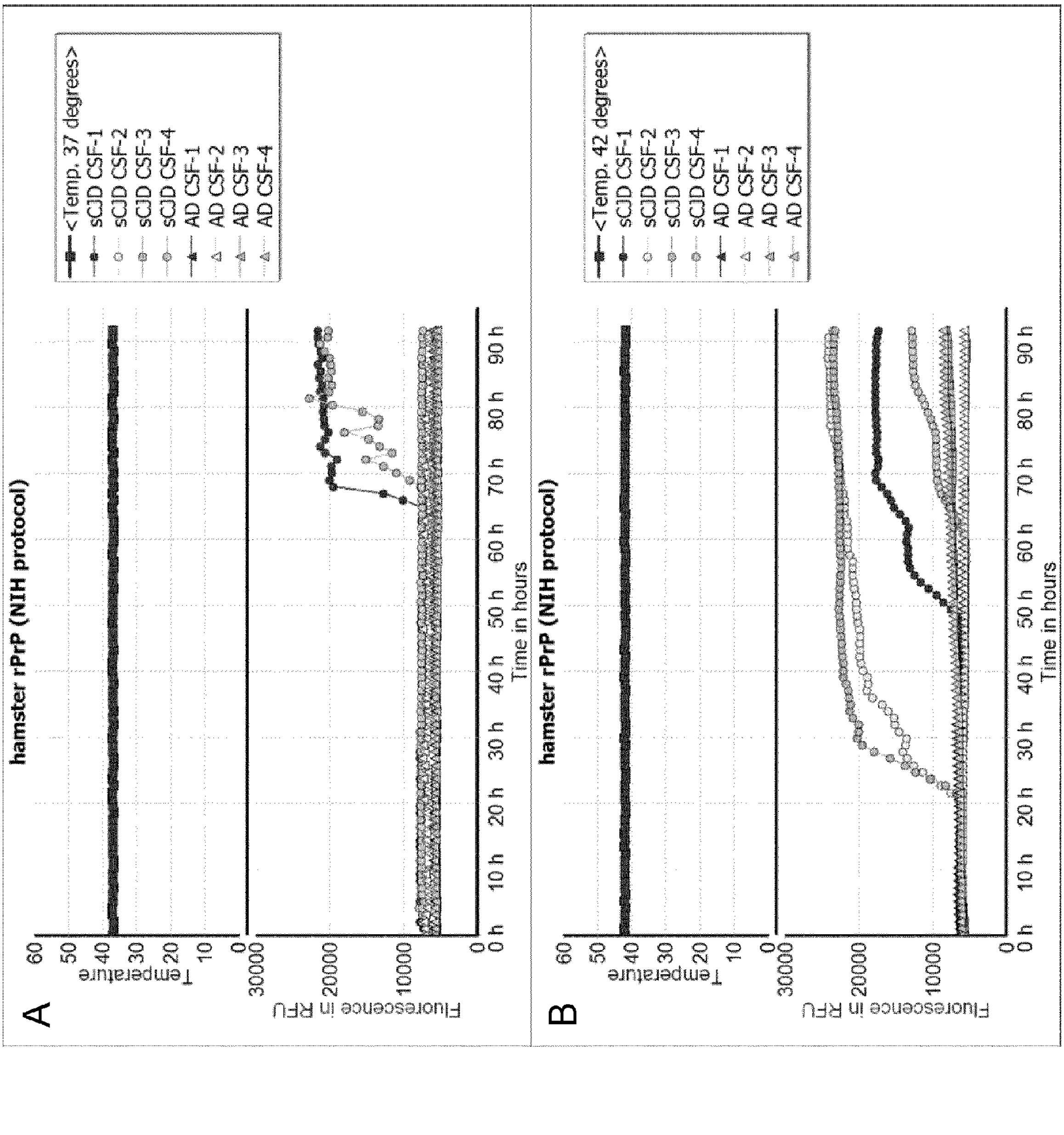
A
hamster rPrP (NIH protocol)
<Temp. 37 degrees>
sCJD CSF-1
sCJD CSF-2
sCJD CSF-3
sCJD CSF-4
AD CSF-1
AD CSF-2
AD CSF-3
AD CSF-4
Temperature
Fluorescence in RFU
Time in hours
B
hamster rPrP (NIH protocol)
<Temp. 42 degrees>
sCJD CSF-1
sCJD CSF-2
sCJD CSF-3
sCJD CSF-4
AD CSF-1
AD CSF-2
AD CSF-3
AD CSF-4
Temperature
Fluorescence in RFU
Time in hours

Figure 1A-B

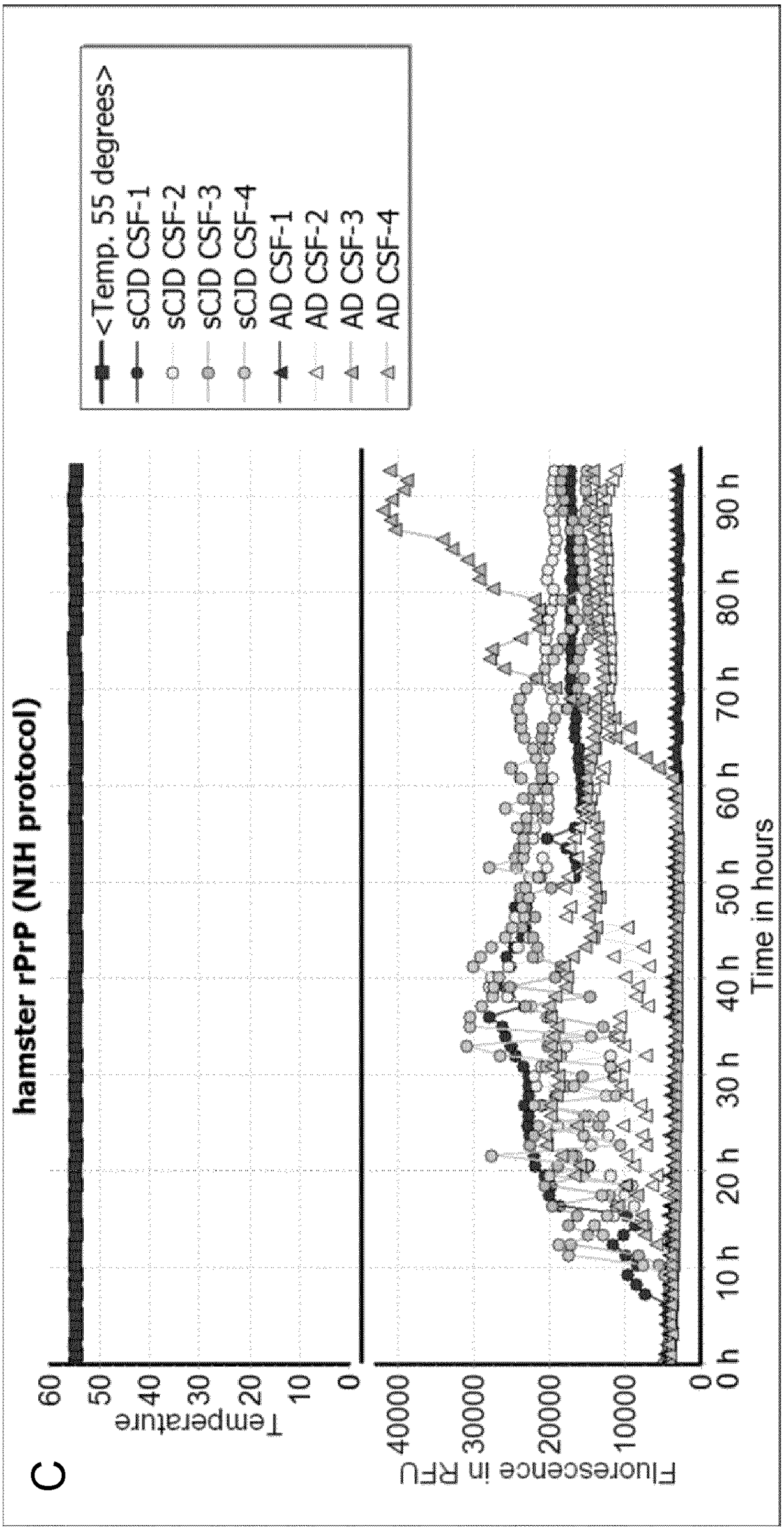
C
hamster rPrP (NIH protocol)
Temperature
60
50
40
30
20
10
0
Fluorescence in RFU
40000
30000
20000
10000
0
0 h
10 h
20 h
30 h
40 h
50 h
60 h
70 h
80 h
90 h
Time in hours
<Temp. 55 degrees>
sCJD CSF-1
sCJD CSF-2
sCJD CSF-3
sCJD CSF-4
AD CSF-1
AD CSF-2
AD CSF-3
AD CSF-4

Figure 1C

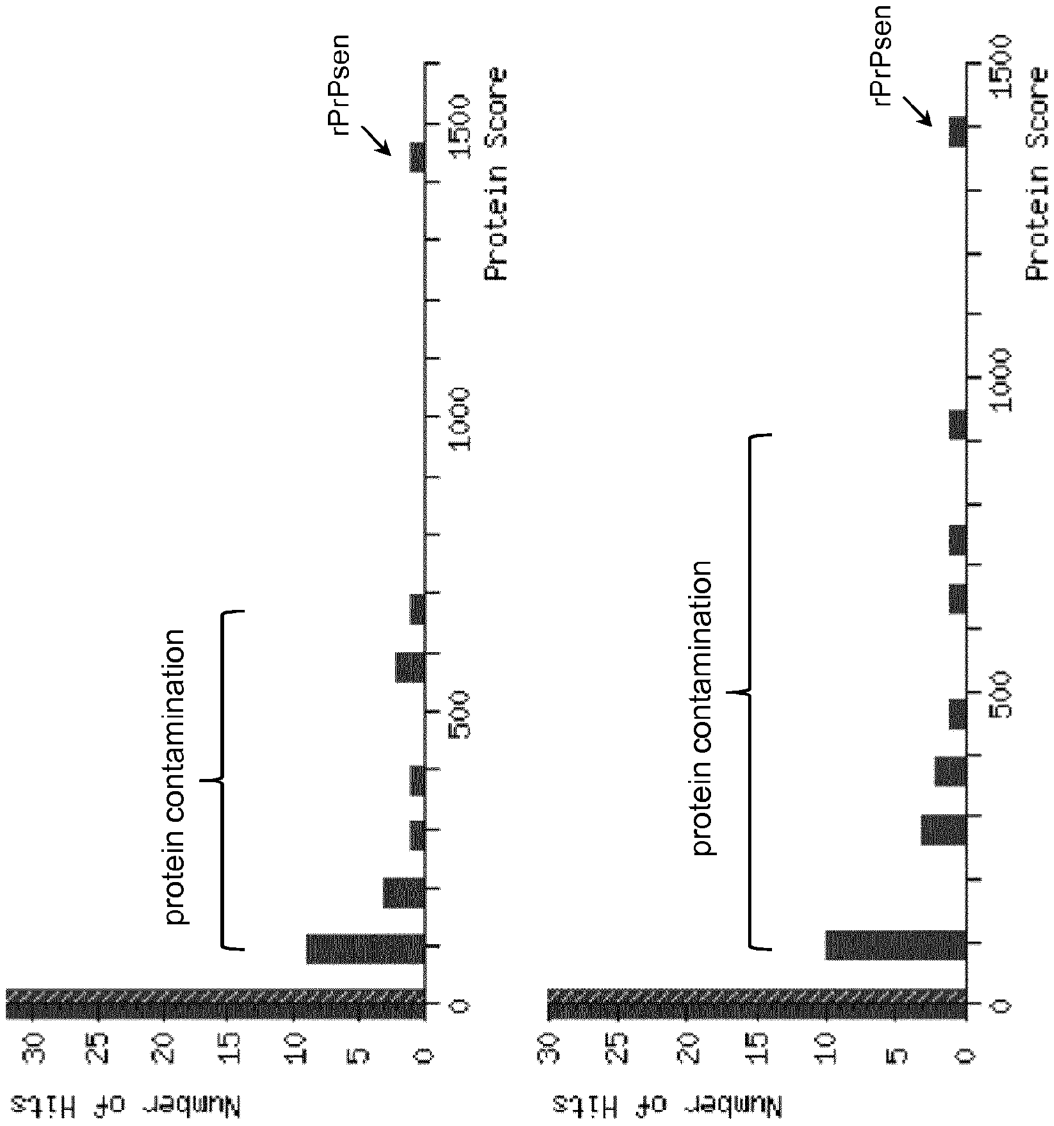
protein contamination
rPrPsen
Protein Score
Number of Hits
0
500
1000
1500
5
10
15
20
25
30
protein contamination
rPrPsen
Protein Score
Number of Hits

**Figure 2**

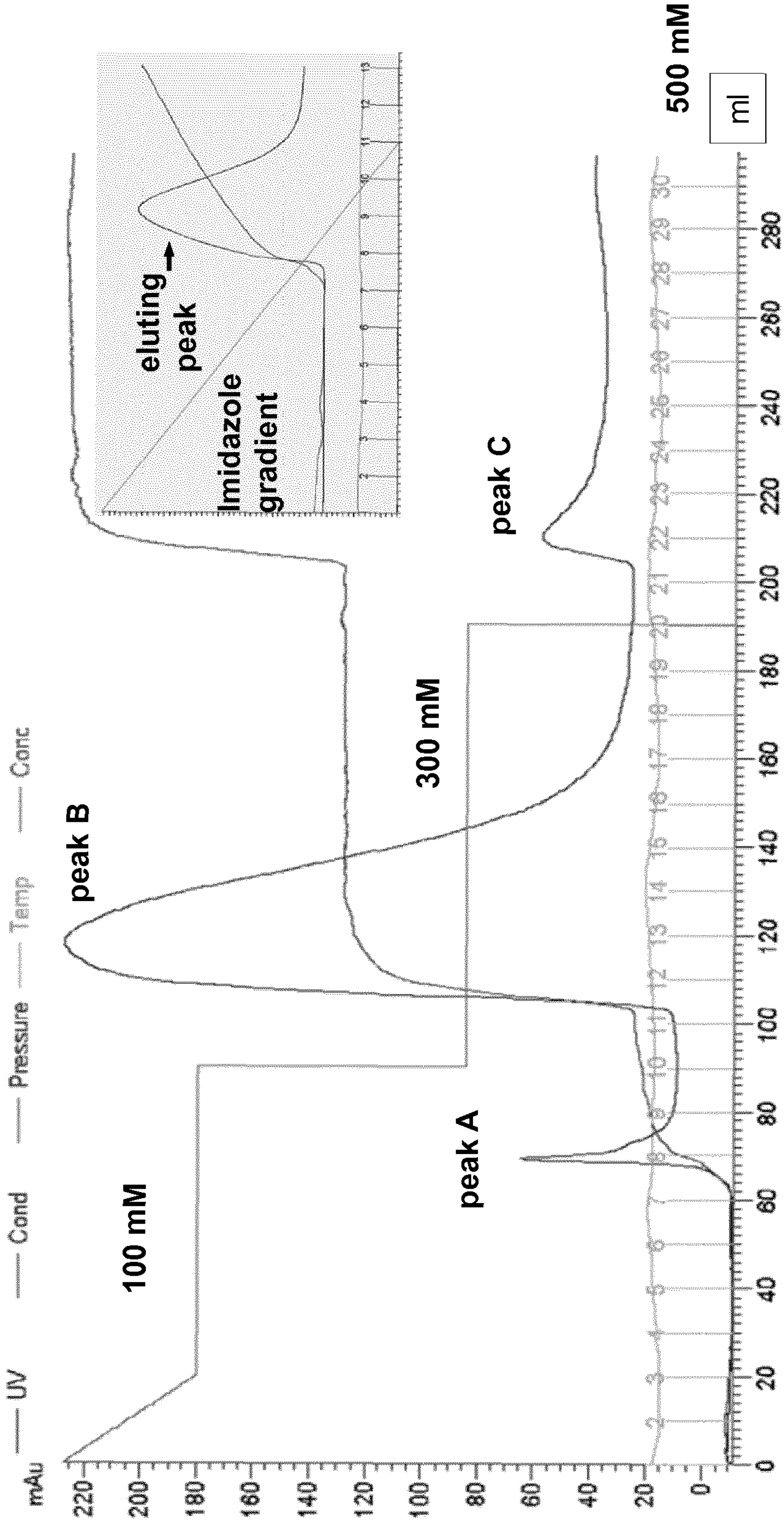
mAu
UV
Cond
Pressure
Temp
Conc
100 mM
300 mM
500 mM
peak A
peak B
peak C
ml
eluting peak
Imidazole gradient

Figure 3

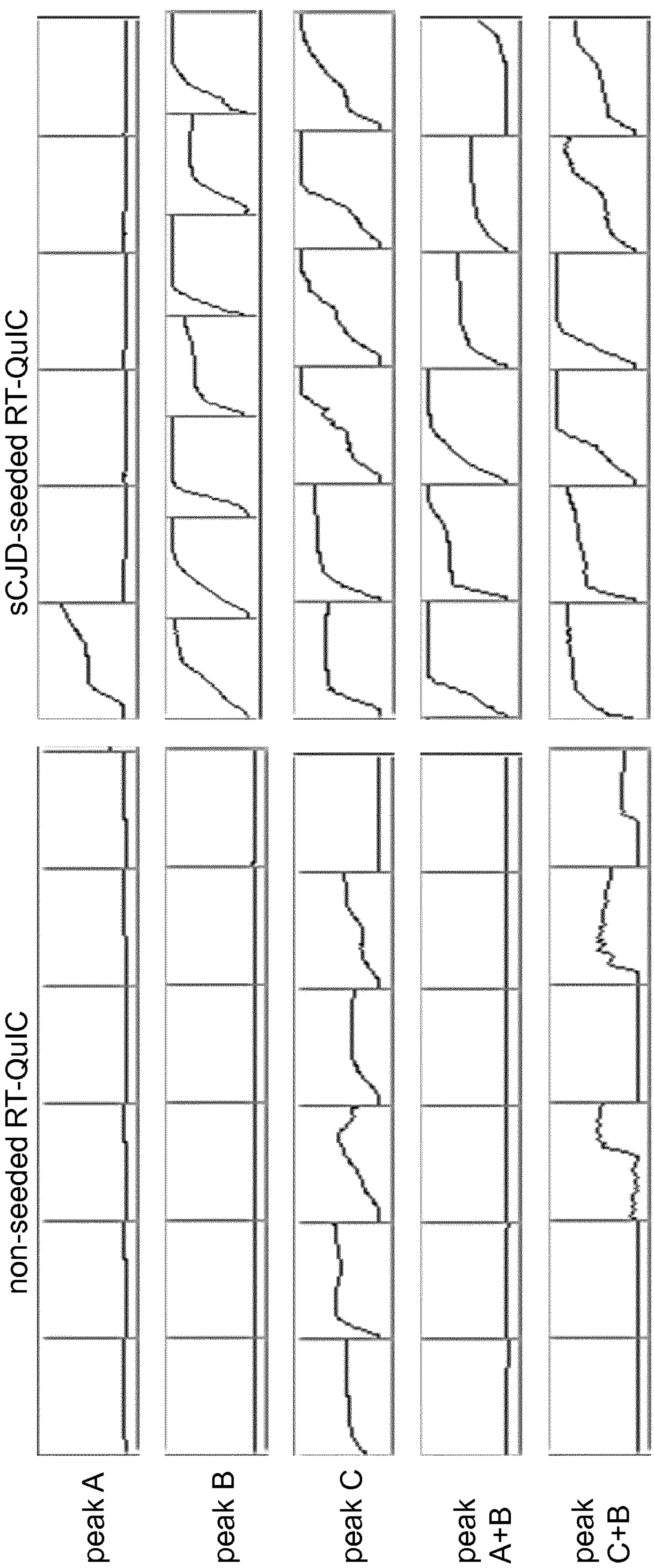

non-seeded RT-QuIC
sCJD-seeded RT-QuIC
peak A
peak B
peak C
peak A+B
peak C+B


Figure 4

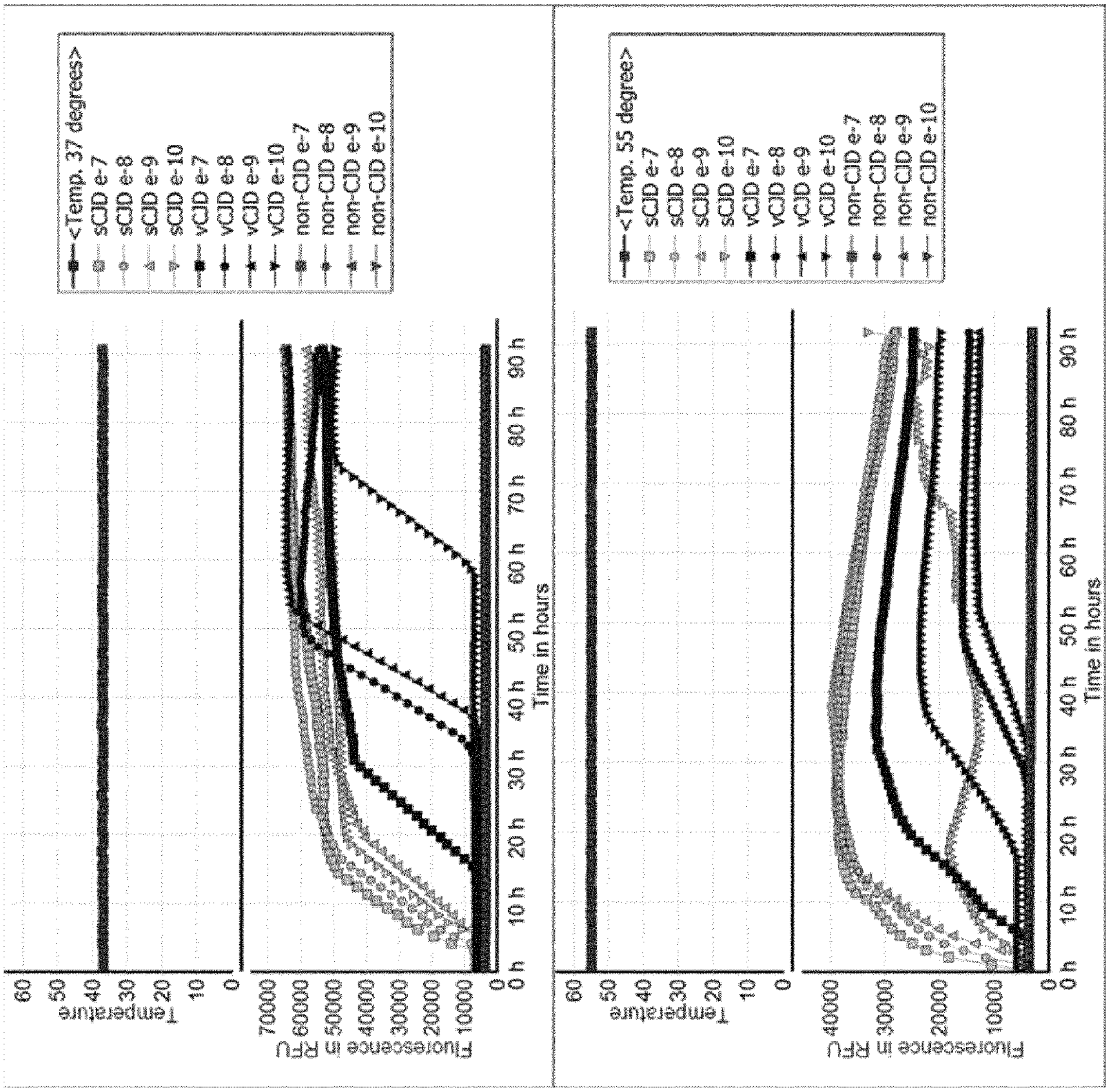
Temperature
60
50
40
30
20
10
0
<Temp. 37 degrees>
sCJD e-7
sCJD e-8
sCJD e-9
sCJD e-10
vCJD e-7
vCJD e-8
vCJD e-9
vCJD e-10
non-CJD e-7
non-CJD e-8
non-CJD e-9
non-CJD e-10
Fluorescence in RFU
70000
60000
50000
40000
30000
20000
10000
0
0 h
10 h
20 h
30 h
40 h
50 h
60 h
70 h
80 h
90 h
Time in hours
<Temp. 55 degree>
40000
30000
20000
10000

Figure 5A

Figure 5B

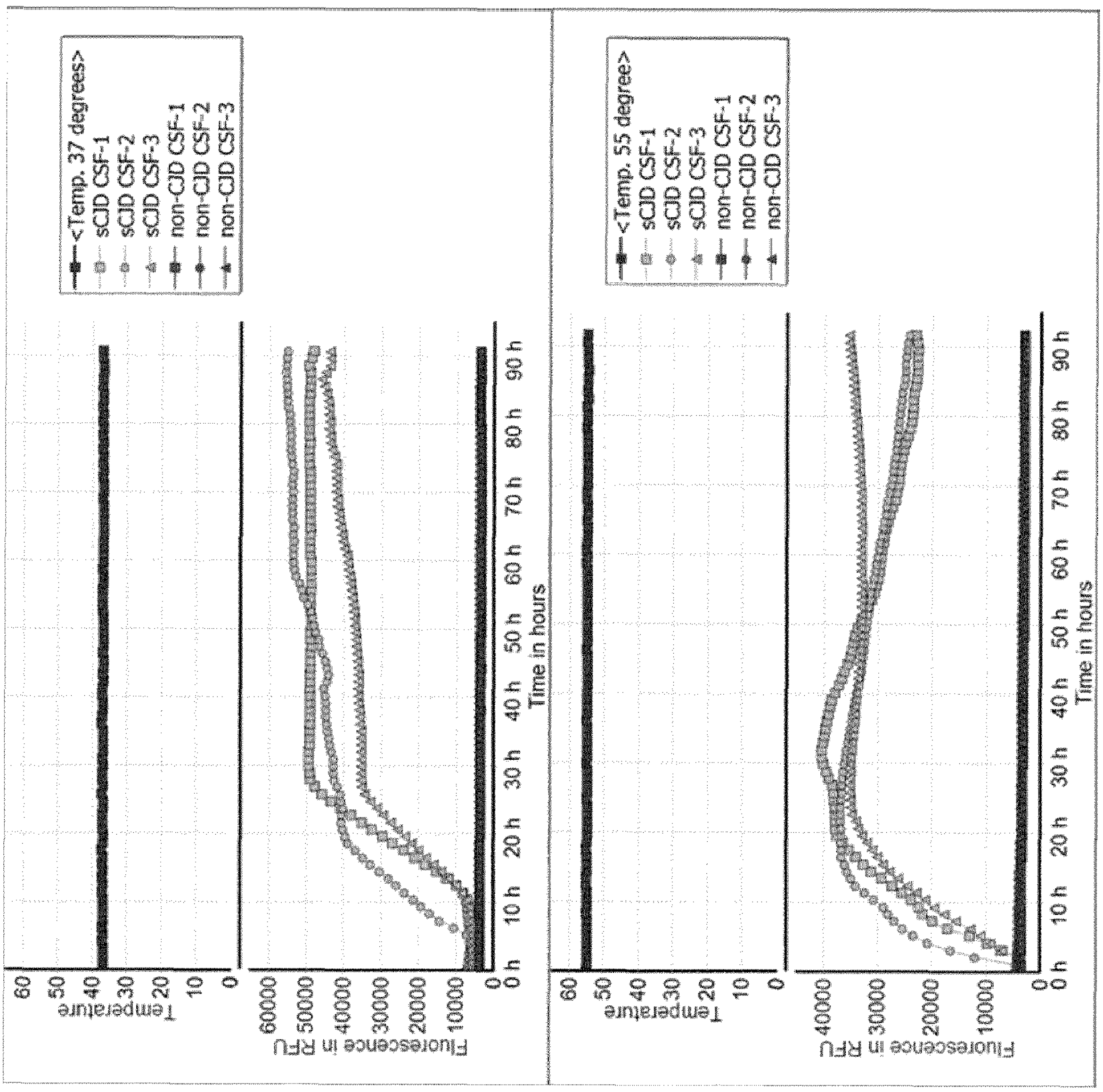
Temperature
Fluorescence in RFU
Time in hours
<Temp. 37 degrees>
sCJD CSF-1
sCJD CSF-2
sCJD CSF-3
non-CJD CSF-1
non-CJD CSF-2
non-CJD CSF-3
<Temp. 55 degree>
0 h
10 h
20 h
30 h
40 h
50 h
60 h
70 h
80 h
90 h

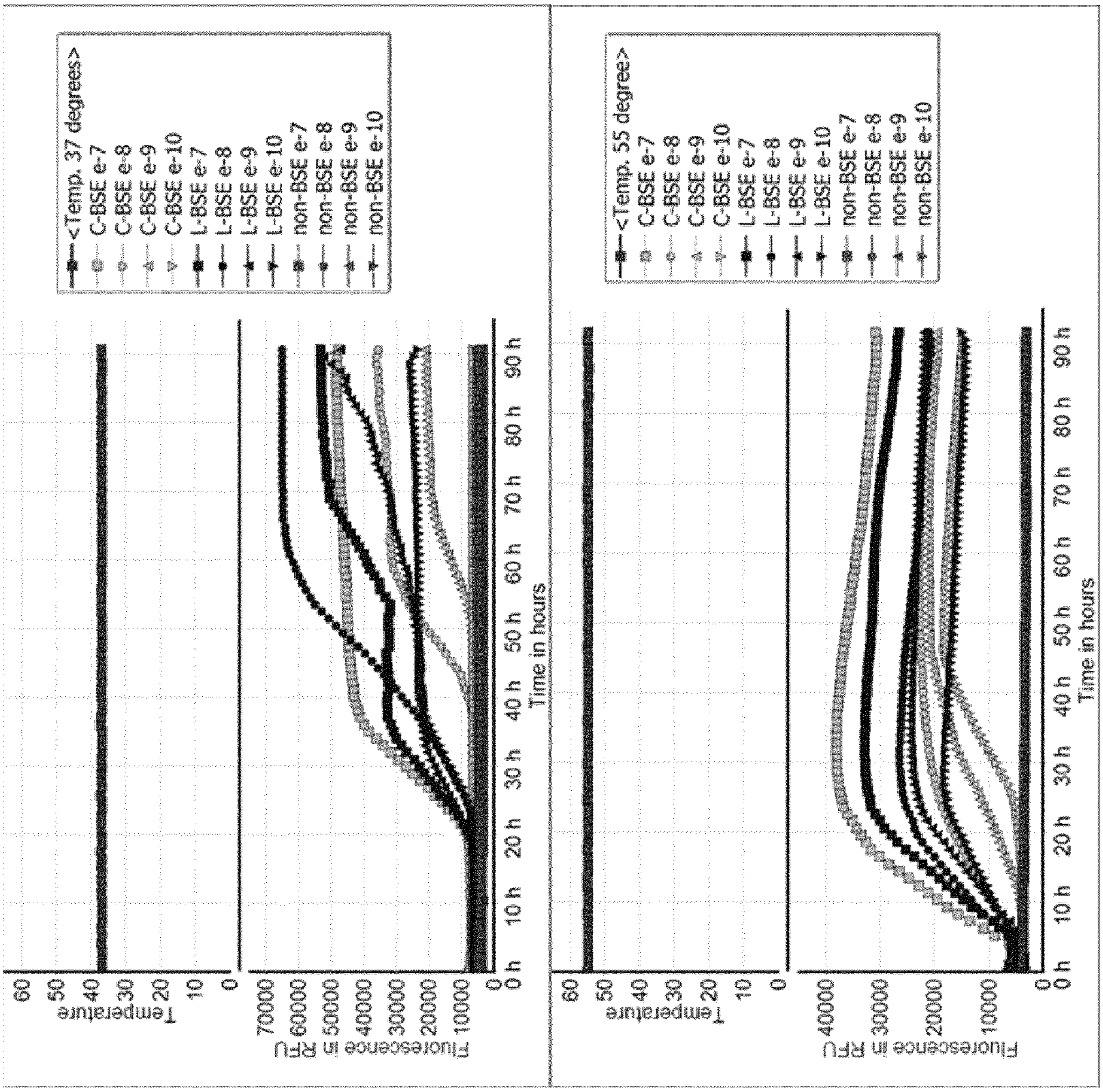
Temperature
Fluorescence in RFU
Time in hours
<Temp. 37 degrees>
C-BSE e-7
C-BSE e-8
C-BSE e-9
C-BSE e-10
L-BSE e-7
L-BSE e-8
L-BSE e-9
L-BSE e-10
non-BSE e-7
non-BSE e-8
non-BSE e-9
non-BSE e-10
<Temp. 55 degree>
C-BSE e-7
C-BSE e-8
C-BSE e-9
C-BSE e-10
L-BSE e-7
L-BSE e-8
L-BSE e-9
L-BSE e-10
non-BSE e-7
non-BSE e-8
non-BSE e-9
non-BSE e-10

Figure 5C

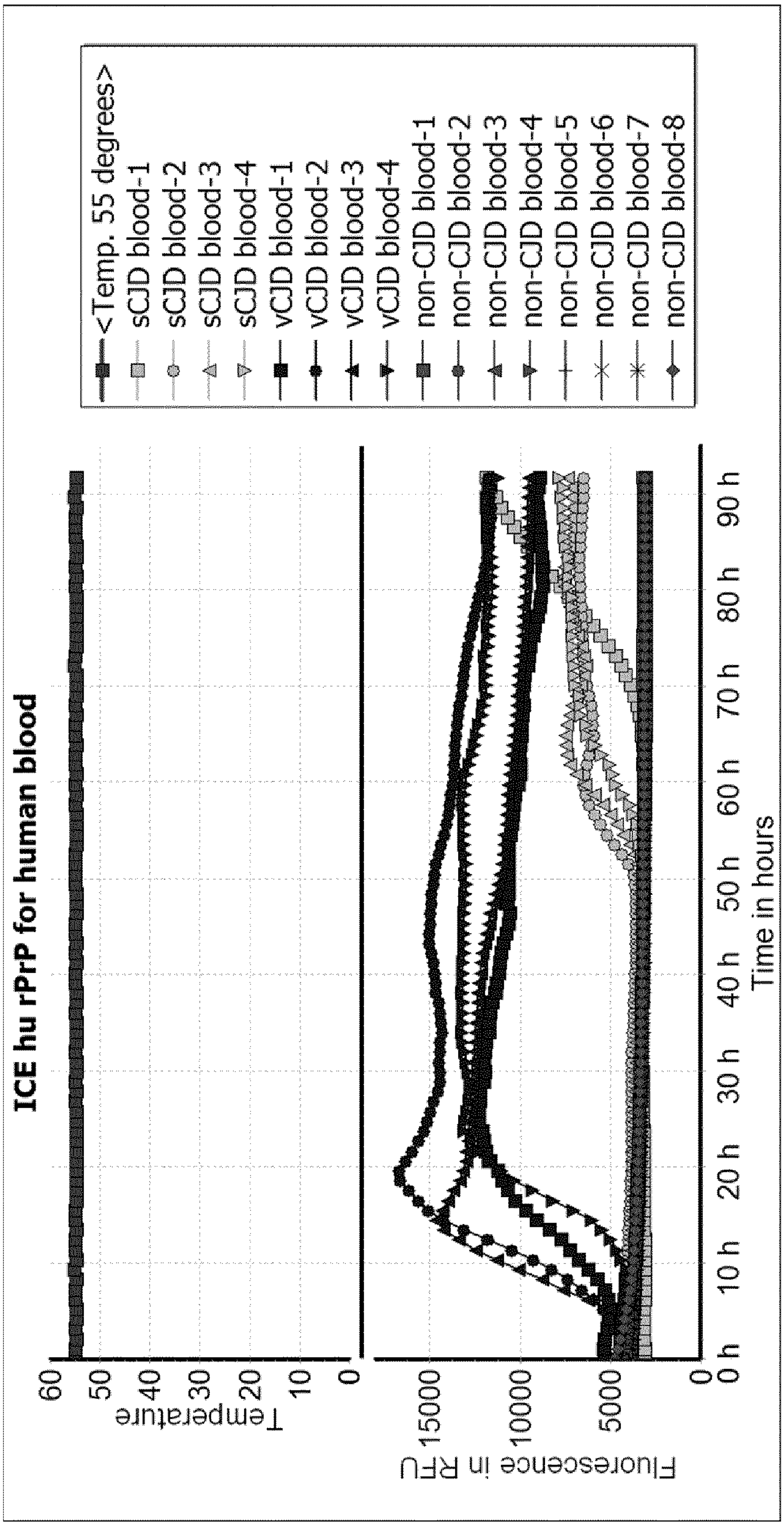
ICE hu rPrP for human blood
Temperature
60
50
40
30
20
10
0
Fluorescence in RFU
15000
10000
5000
0
0 h
10 h
20 h
30 h
40 h
50 h
60 h
70 h
80 h
90 h
Time in hours
<Temp. 55 degrees>
sCJD blood-1
sCJD blood-2
sCJD blood-3
sCJD blood-4
vCJD blood-1
vCJD blood-2
vCJD blood-3
vCJD blood-4
non-CJD blood-1
non-CJD blood-2
non-CJD blood-3
non-CJD blood-4
non-CJD blood-5
non-CJD blood-6
non-CJD blood-7
non-CJD blood-8

**Figure 6**

Figure 7.1

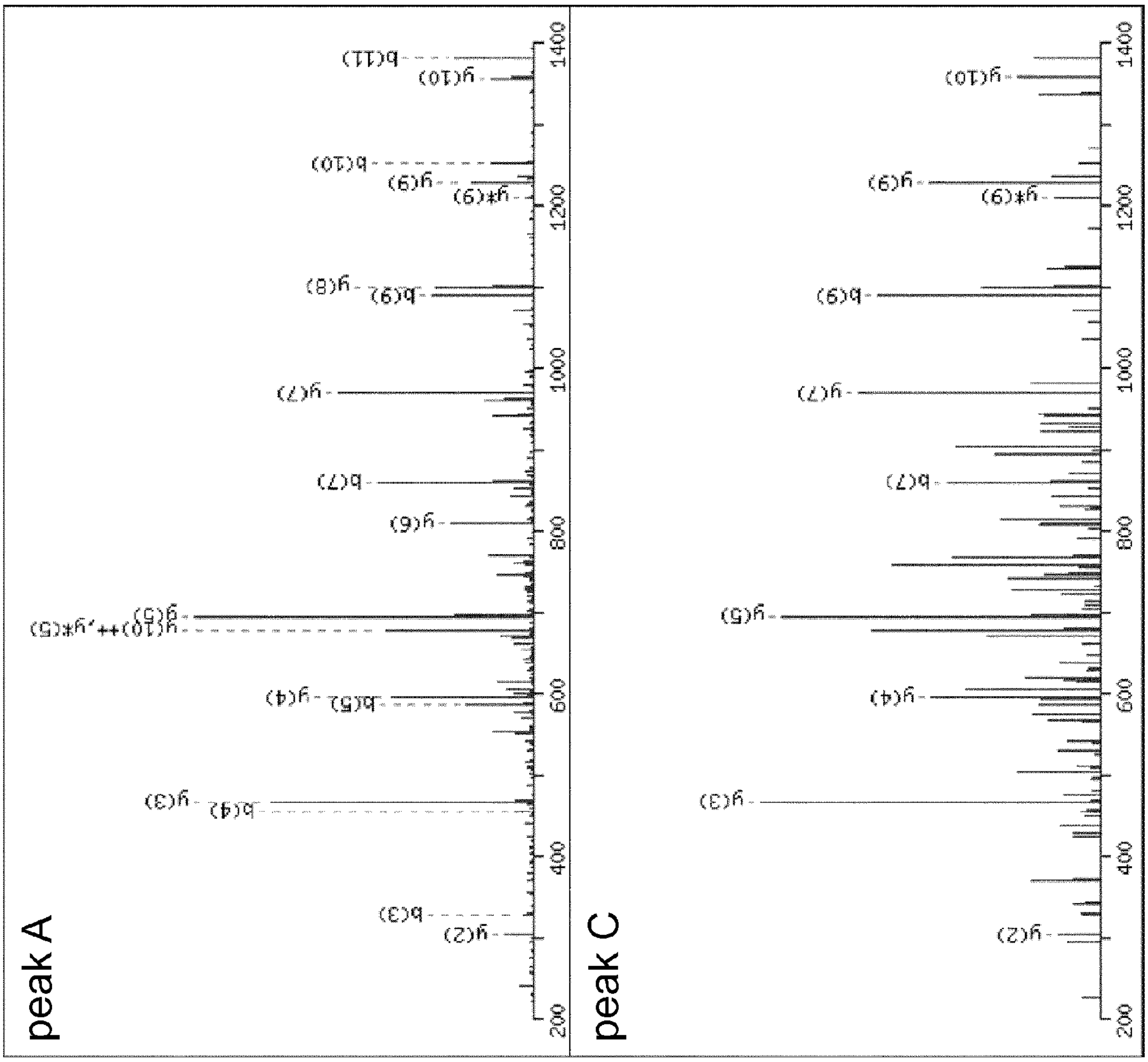
peak A
peak C
y(2)
b(3)
b(4)
y(3)
b(5)
y(4)
y(10)++, y*(5)
y(5)
y(6)
b(7)
y(7)
b(9)
y(8)
y*(9)
y(9)
b(10)
y(10)
b(11)
200
400
600
800
1000
1200
1400

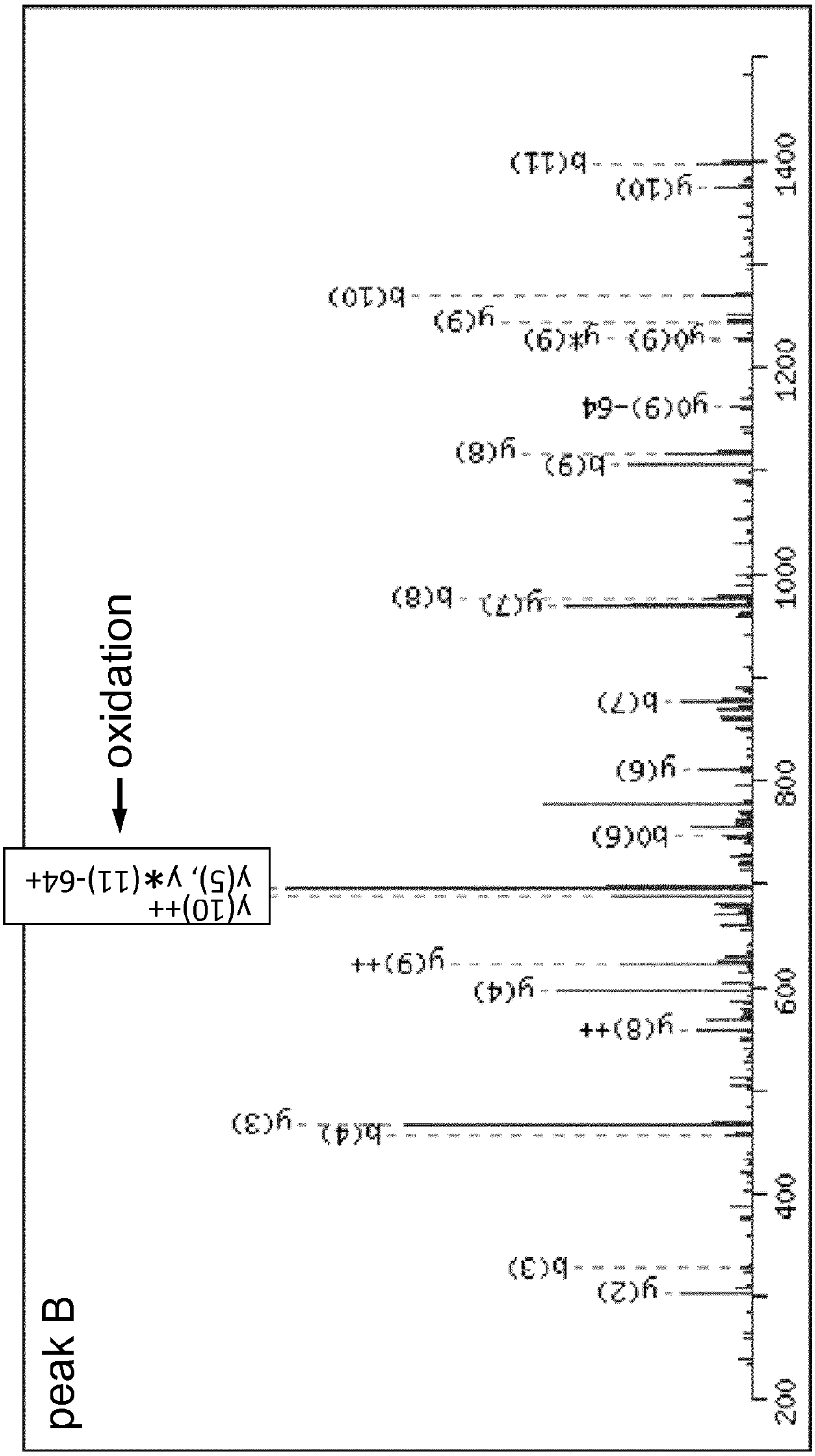
peak B
oxidation
y(10)++
y(5), y*(11)-64+
y(2)
b(3)
b(4)
y(3)
y(8)++
y(4)
y(9)++
b0(6)
y(6)
b(7)
y(7)
b(8)
b(9)
y(8)
y0(9)-64
y0(9)
y*(9)
y(9)
b(10)
y(10)
b(11)
200
400
600
800
1000
1200
1400

Figure 7.2

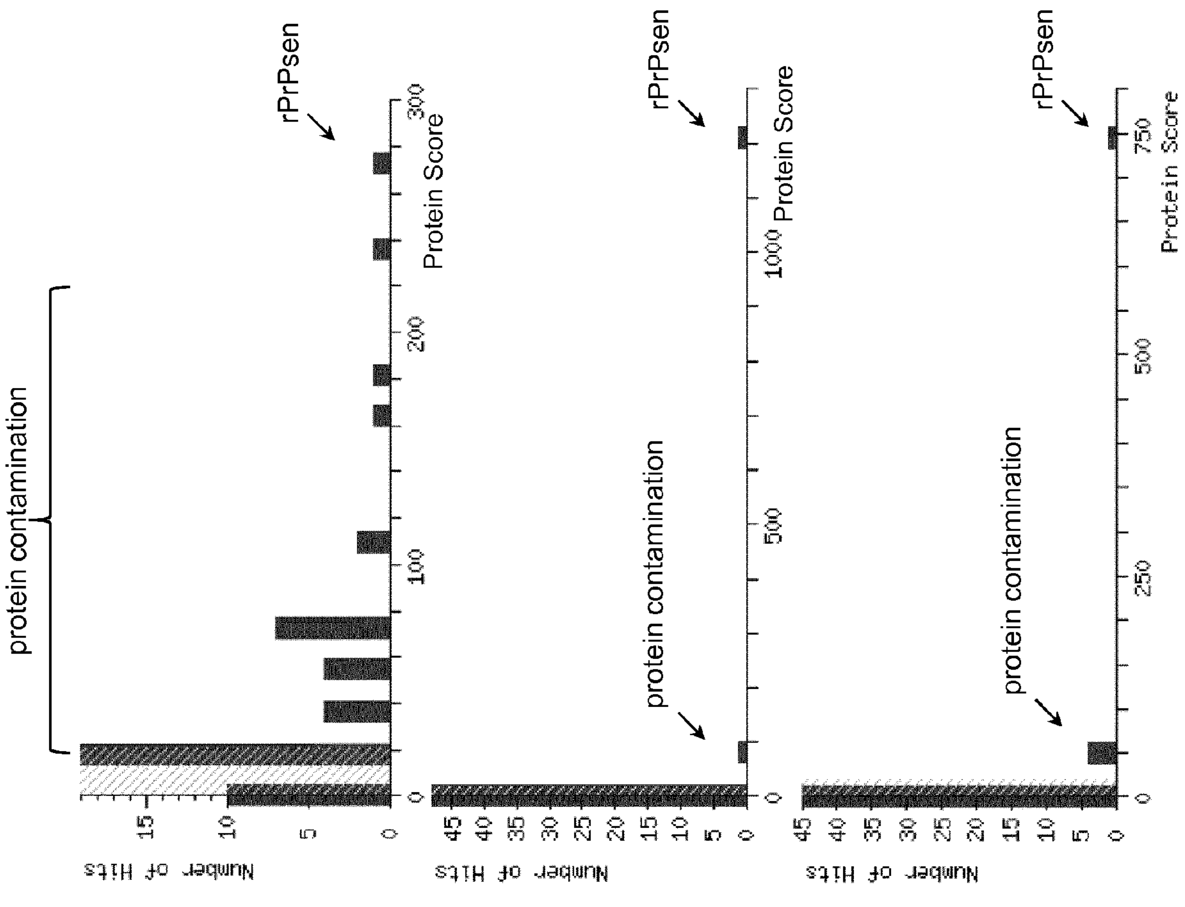
protein contamination
rPrPsen
Protein Score
Number of Hits
protein contamination
rPrPsen
Protein Score
Number of Hits
protein contamination
rPrPsen
Protein Score
Number of Hits

**Figure 8**

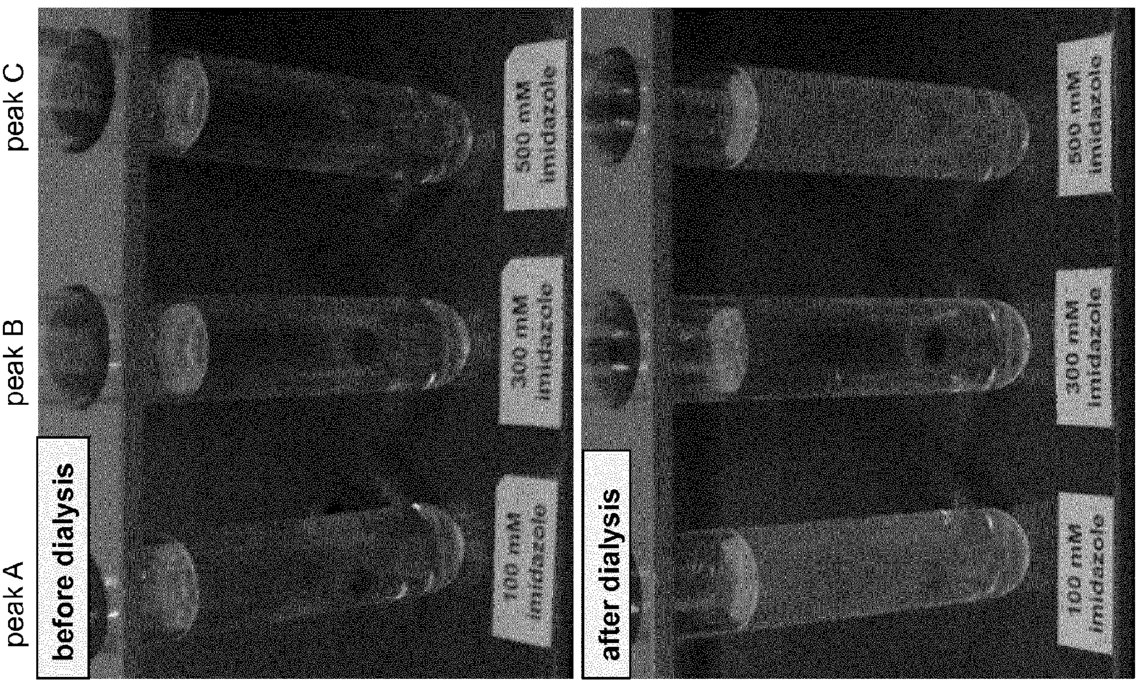
peak A
peak B
peak C
before dialysis
100 mM imidazole
300 mM imidazole
500 mM imidazole
after dialysis
100 mM imidazole
300 mM imidazole
500 mM imidazole

Figure 9

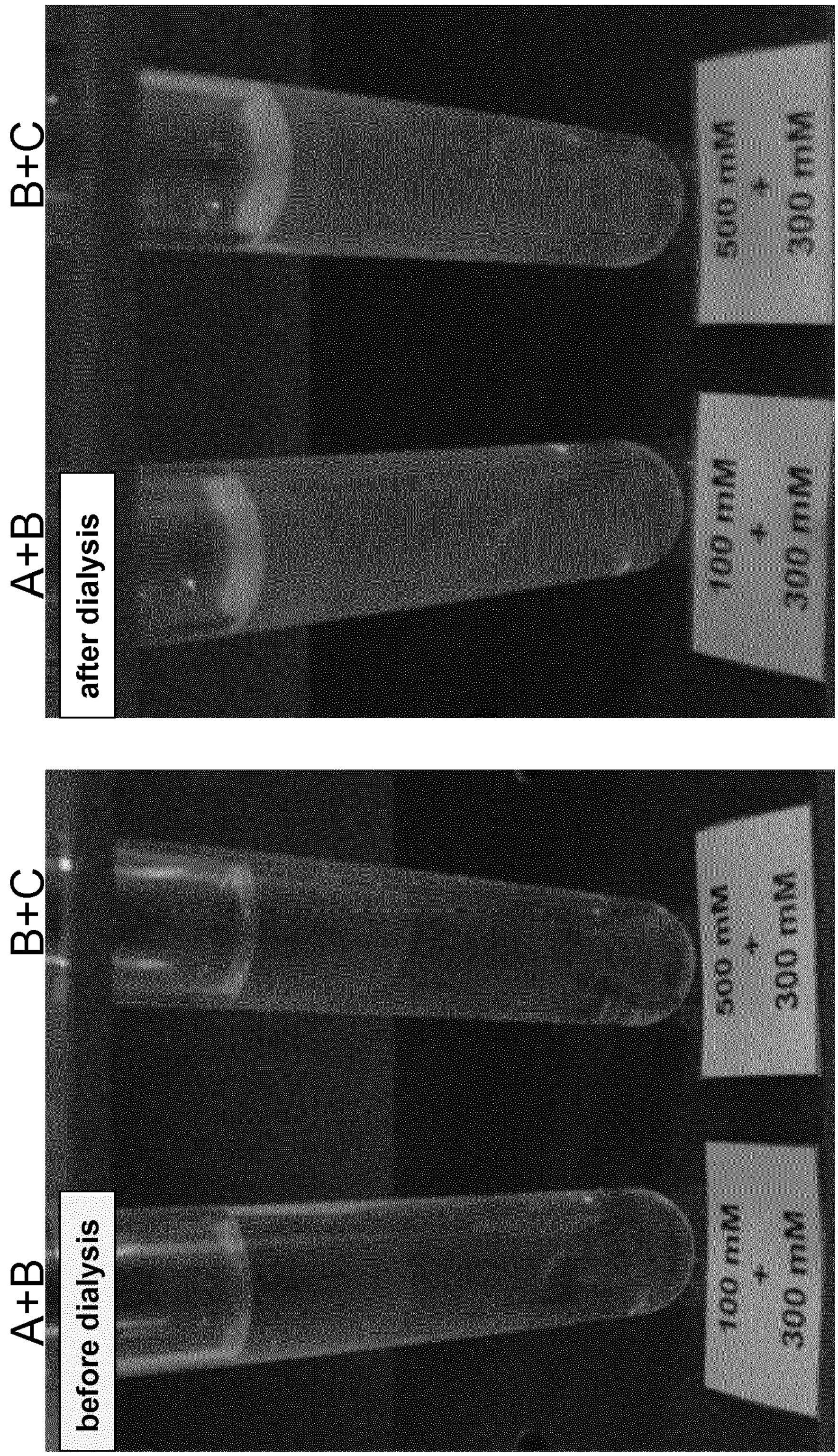
A+B
B+C
before dialysis
100 mM
+
300 mM
500 mM
+
300 mM
A+B
B+C
after dialysis
100 mM
+
300 mM
500 mM
+
300 mM

Figure 10

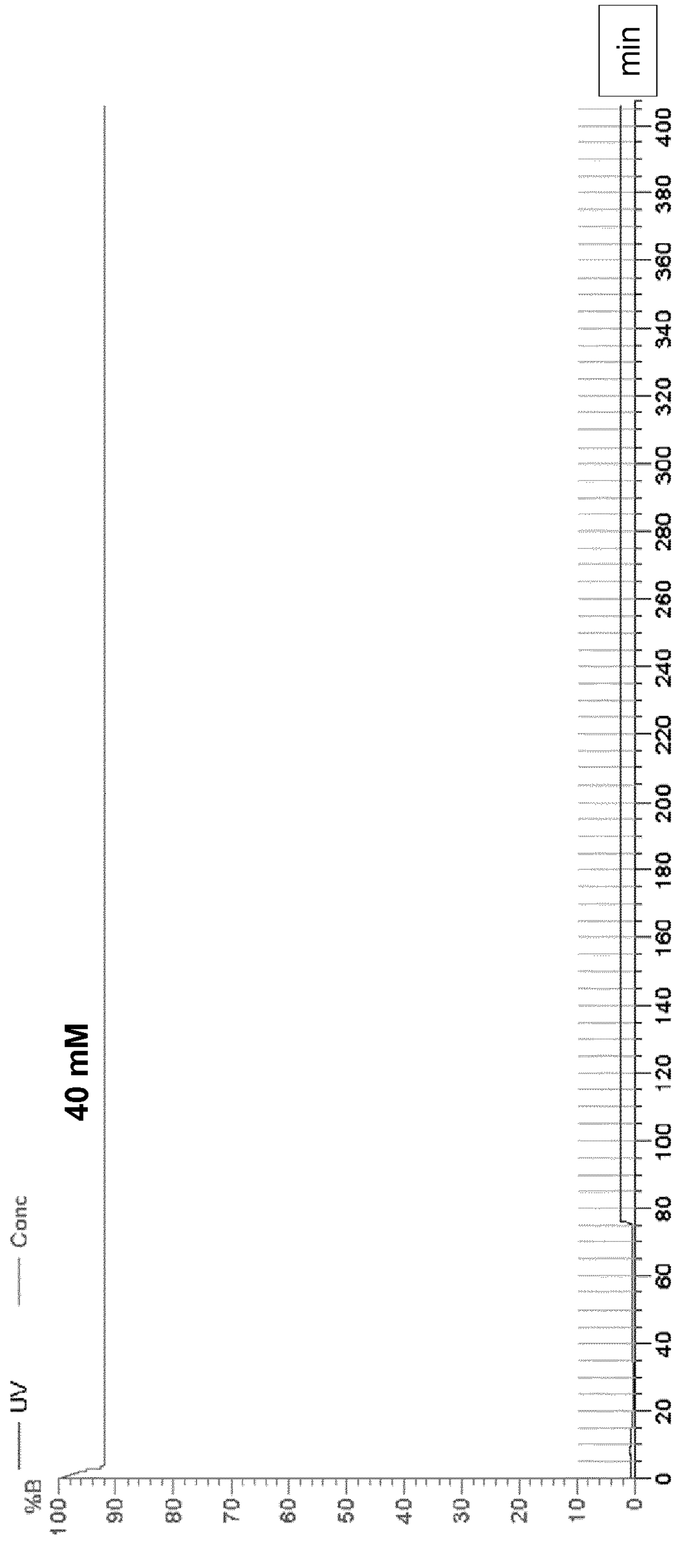
40 mM
UV
Conc
%B
min
100
90
80
70
60
50
40
30
20
10
0
0
20
40
60
80
100
120
140
160
180
200
220
240
260
280
300
320
340
360
380
400

Figure 11

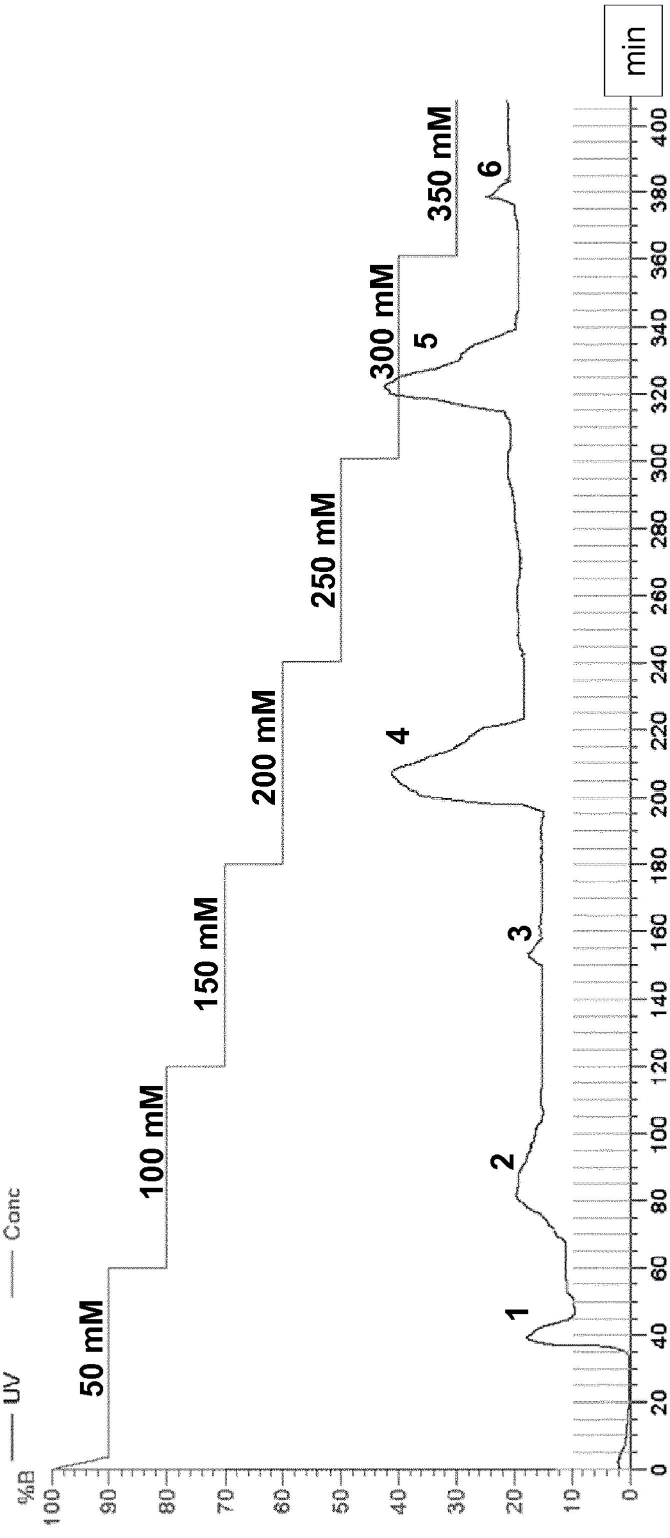
UV
Conc
%B
100
90
80
70
60
50
40
30
20
10
0
50 mM
100 mM
150 mM
200 mM
250 mM
300 mM
350 mM
1
2
3
4
5
6
0
20
40
60
80
100
120
140
160
180
200
220
240
260
280
300
320
340
360
380
400
min

Figure 12

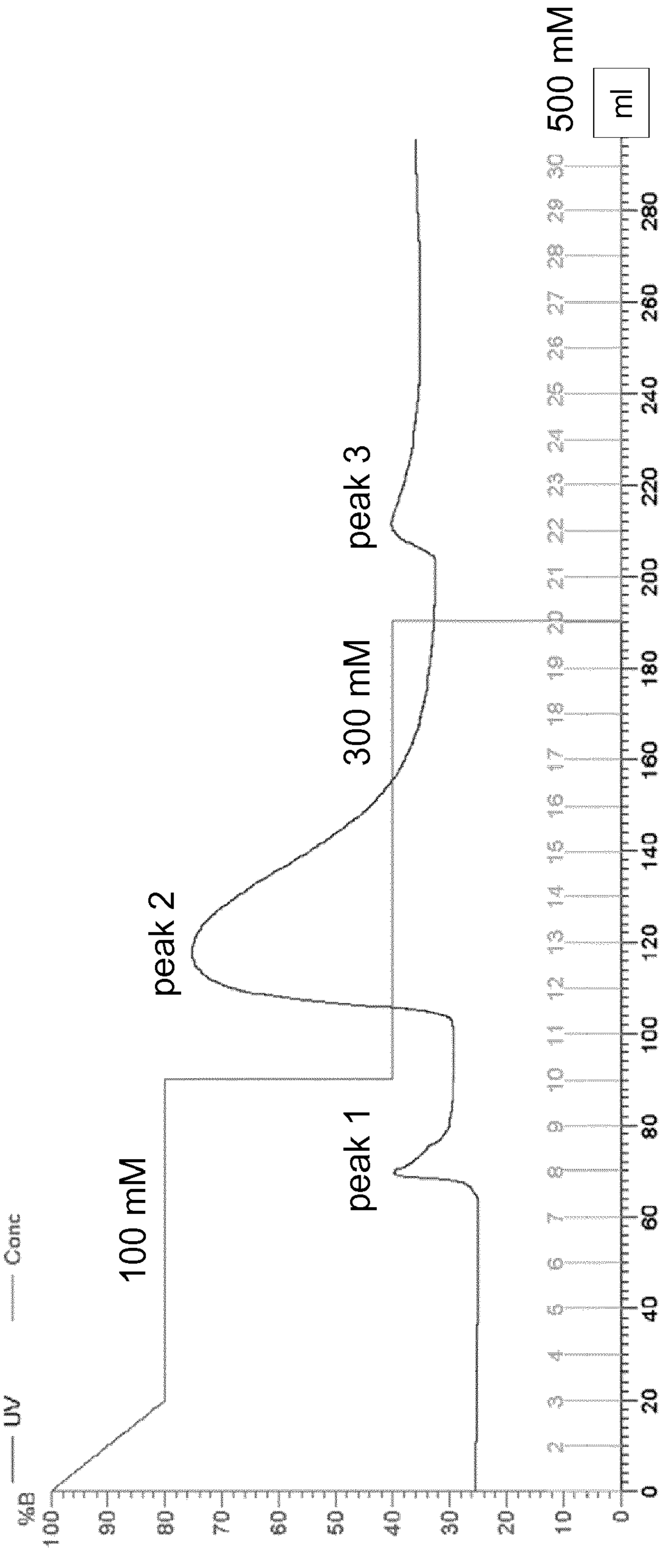


**Figure 13**

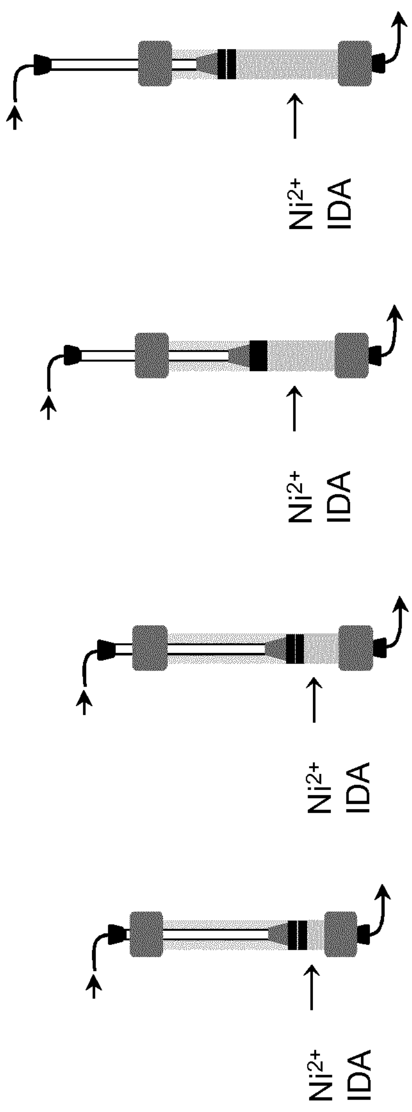


Figure 14

Europäisches Patentamt
European Patent Office
Office européen des brevets

# EUROPEAN SEARCH REPORT

Application Number
EP 13 18 2421

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 102 887 949 A (UNIV CHINA AGRICULTURAL) 23 January 2013 (2013-01-23)<br>* paragraph [0082] - paragraph [0083] *<br>----- | 1-16 | INV.<br>C07K1/22<br>G01N33/68 |
| X | WO 2008/083972 A2 (ALICON AG [CH]; ZAHN RALPH [CH]; EL GEDAILY AHMED [CH]; FRANSCINI NICO) 17 July 2008 (2008-07-17)<br>* the whole document *<br>----- | 1-16 | |
| X | US 2006/030007 A1 (BYRD DEVON R [US] ET AL) 9 February 2006 (2006-02-09)<br>* the whole document *<br>----- | 1-16 | |
| X | EP 1 674 870 A2 (ROCHE DIAGNOSTICS GMBH [DE]; HOFFMANN LA ROCHE [CH]) 28 June 2006 (2006-06-28)<br>* the whole document *<br>----- | 1-16 | |
| A | ATARASHI R ET AL: "Simplified ultrasensitive prion detection by recombinant PrP conversion with shaking", NATURE METHODS, NATURE PUBLISHING GROUP, GB,<br>vol. 5, no. 3, 1 March 2008 (2008-03-01), pages 211-212, XP002656329,<br>ISSN: 1548-7091<br>* the whole document *<br>----- | 1-16 | TECHNICAL FIELDS SEARCHED (IPC)<br>C07K<br>G01N |

The present search report has been drawn up for all claims

1

| Place of search | Date of completion of the search | Examiner |
|---|---|---|
| Munich | 15 October 2013 | Seranski, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 18 2421

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2013

| Patent document cited in search report | Publication date | Patent family member(s) | Publication date |
|---|---|---|---|
| CN 102887949 A | 23-01-2013 | NONE | |
| WO 2008083972 A2 | 17-07-2008 | CA 2675039 A1 | 17-07-2008 |
| | | EP 2122367 A2 | 25-11-2009 |
| | | JP 2010515879 A | 13-05-2010 |
| | | US 2009258419 A1 | 15-10-2009 |
| | | WO 2008083972 A2 | 17-07-2008 |
| US 2006030007 A1 | 09-02-2006 | US 2006030007 A1 | 09-02-2006 |
| | | WO 2004106361 A2 | 09-12-2004 |
| EP 1674870 A2 | 28-06-2006 | AT 389182 T | 15-03-2008 |
| | | AT 398288 T | 15-07-2008 |
| | | CA 2523745 A1 | 15-05-2006 |
| | | DE 602005005284 T2 | 16-04-2009 |
| | | EP 1674870 A2 | 28-06-2006 |
| | | EP 1677114 A1 | 05-07-2006 |
| | | EP 1677115 A2 | 05-07-2006 |
| | | US 2006121507 A1 | 08-06-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ATARASHI R ; WILHAM JM ; CHRISTENSEN L et al.** *Nat Methods,* 2008, vol. 5, 211-212
- **WILHAM JM et al.** *PLoS,* 2010, vol. 6 (12
- **ATARASHI R et al.** *Nature Medicine,* 2011, vol. 17 (1
- **ATARASHI et al.** *Prion,* 2011, vol. 5, 150-153
- **ORRU et al.** *Protein Eng Des Sel.,* 2009, vol. 22, 515-521
- **COLOMBO et al.** *PLoS one,* 2009, vol. 4, e4296
- **MELI et al.** *PLoS one,* 2011, vol. 6, e19093
- **WOLSCHNER et al.** *Proc Natl Acad Sci U S A.,* 2009, vol. 106, 7756-7761
- **PEDEN et al.** *J. Gen. Virol,* 2012, vol. 93, 438-449
- **MCGUIRE et al.** *Prion,* 2011, vol. 5, 18
- **ATARASHI R ; WILHAM JM ; CHRISTENSEN L et al.** Simplified ultrasensitive detection of recombinant PrP conversion with shaking. *Nat Methods,* 2008, vol. 5, 211-212
- **WILHAM JM ; ORRU CD ; BESSEN RA et al.** Rapid end-point quantitation of prion seeding activity with sensitivity comparable to bioassays. *PLoS,* 2010, vol. 6 (12
- **ATARASHI R ; SATOH K ; SANO K et al.** Ultrasensitive human prion detection in cerebrospinal fluid by real-time quaking induced conversion. *Nature Medicine,* 2011, vol. 17 (1
- **ATARASHI R ; SANO K ; SATOH K et al.** Real-time quaking-induced conversion: a highly sensitive assay for prion detection. *Prion,* 2011, vol. 5, 150-153
- **ORRU CD ; WILHAM JM ; HUGHSON AG et al.** Human variant Creutzfeldt-Jakob disease and sheep scrapie PrP(res) detection using seeded conversion of recombinant prion protein. *Protein Eng Des Sel.,* 2009, vol. 22, 515-521
- **COLOMBO G ; MELI M ; MORRA G ; GABIZON R ; GASSET M.** Methionine sulfoxides on prion protein Helix-3 switch on the alpha-fold destabilization required for conversion. *PLoS one,* 2009, vol. 4, e4296
- **MELI M ; GASSET M ; COLOMBO G.** Dynamic Diagnosis of Familial Prion Disease Supports the $\beta$2-$\alpha$2 Loop as a Universal Interference Target. *PLoS one,* 2011, vol. 6, e19093
- **WOLSCHNER C ; GIESE A ; KRETZSCHMAR HA et al.** Design of anti- and pro-aggregation variants to assess the effects of methionine oxidation in human prion protein. *Proc Natl Acad Sci U S A.,* 2009, vol. 106, 7756-7761
- **PEDEN AH ; MCGUIRE LI ; APPLEFORD NE et al.** Sensitive and specific detection of sporadic Creutzfeldt-Jakob disease brain prion protein using real-time quaking-induced conversion. *J. Gen. Virol,* 2012, vol. 93, 438-449
- **MCGUIRE L ; PEDEN A ; APPLEFORD N et al.** Prion seeding activity in cerebrospinal fluid from sporadic Creutzfeldt-Jakob disease patients using real-time QuIC analysis: a potential new diagnostic test?. *Prion,* 2011, vol. 5, 18